# EUROPEAN PATENT APPLICATION

(11) **EP 4 585 680 A1**
(43) Date of publication of application: **16.07.2025**
(21) Application number: 23863222.8
(22) Date of filing: 06.09.2023
(51) Int. Cl.: C12N 5/071

(54) **FEEDER CELLS, CELL SHEET, PRODUCTION METHOD FOR FEEDER CELLS AND CELL SHEET, AND METHOD FOR MAINTAINING OR PROLIFERATING CELLS USING FEEDER CELL**

(30) Priority: 06.09.2022 JP 2022141462
(71) Applicant: Dai Nippon Printing Co., Ltd., Tokyo 162-8001 (JP); National Center for Child Health and Development, Tokyo 157-8535 (JP)
(72) Inventor: MORINAGA Kenichi, Tokyo 162-8001 (JP); UMEZAWA Akihiro, Tokyo 157-8535 (JP)
(74) Representative: Beck Greener LLP
(86) International application number: PCT/JP2023/032577
(87) International publication number: WO 2024/053684

(57) **Abstract**

[Problem] Provided are: a feeder cell for maintaining or proliferating, by a two-dimensional culture, a cell deemed to be difficult to maintain and proliferate by a two-dimensional culture; and a method of producing the feeder cell.

[Solution] A fibroblast-like cell is produced from a pluripotent stem cell, and derived from an intestinal structure containing an endoderm-derived cell and a mesoderm-derived cell is proliferated, isolated, and used as a feeder cell.

## Description

### Technical Field

The present disclosure relates to a feeder cell for maintaining or proliferating a cell, a cell sheet containing the feeder cell, a method of producing each of the feeder cell and the cell sheet, and a method of maintaining or proliferating a cell using the feeder cell.

### Background Art

In recent years, a primary cultured cell obtained from an organism, pluripotent stem cells such as an embryonic stem cell (ES cell) and an induced pluripotent stem cell (iPS cell), a cell induced to differentiate from a pluripotent stem cell, and the like are widely used in fields such as basic research, regenerative medicine, and drug development.

In these fields, there is a demand for a technology in which a cell having a specific property is proliferated through induction of differentiation from a pluripotent stem cell, or in which a plurality of cells having such a specific property are organized to form a tissue or an organ. Conventionally, such a technology has been studied actively. For example, in a technology disclosed (for example, Patent Literature 1), an intestinal epithelial cell is induced to differentiate from a pluripotent stem cell, and the intestinal epithelial cell after differentiation is further cultured three-dimensionally to be maintained and proliferated, thus forming an intestinal structure.

On the other hand, various attempts have been made at a two-dimensional culture of an intestinal epithelial cell, but a two-dimensional culture method of maintaining and proliferating an intestinal epithelial cell has not been discovered yet.

By the way, there are some cases in which a cell that produces and supplies a specific factor (for example, a differentiation-inducing factor), i.e., a feeder cell is used to induce a cell having a specific property to differentiate from a pluripotent stem cell, or used to promote the maintenance and proliferation of a cell obtained by induction of differentiation (for example, Patent Literature 2).

However, there has not been a known technology in which even an intestinal epithelial cell cultured two-dimensionally is maintained or proliferated using a feeder cell. Accordingly, there is a demand for discovering a practical method of maintaining or proliferating, by a two-dimensional culture, a cell hitherto deemed to be difficult to maintain and proliferate by a two-dimensional culture, examples of which cell include an intestinal epithelial cell.

### Citation List

### Patent Literature

Patent Literature 1: JP2017-184749A
Patent Literature 2: JP2022-7610A

### Summary of Invention

### Technical Problem

Under such a situation, there is a demand for discovering a practical method of maintaining or proliferating, by a two-dimensional culture, a cell hitherto deemed to be difficult to maintain and proliferate by a two-dimensional culture, examples of which cell include an intestinal epithelial cell.

Accordingly, the present disclosure provides: a feeder cell for maintaining or proliferating, by a two-dimensional culture, a cell deemed to be difficult to maintain and proliferate by a two-dimensional culture, examples of which cell include an intestinal epithelial cell; and a method of producing the feeder cell. In addition, the present disclosure provides a cell sheet containing such a feeder cell as described above.

### Solution to Problem

Embodiments of the present disclosure relate to the following [1] to [28].

[1] A method of producing a feeder cell for maintaining or proliferating a cell, including:
   (A) a step of preparing an intestinal structure produced from a pluripotent stem cell, and containing an endoderm-derived cell and a mesoderm-derived cell;
   (B) a step of culturing the intestinal structure to proliferate a fibroblast-like cell derived from the intestinal structure; and
   (C) a step of isolating the fibroblast-like cell to obtain a feeder cell.
[2] The method according to [1], wherein the intestinal structure is produced using a method including:
   (a) a step of preparing a cell culture substrate including: a substrate; and a plurality of isolated cell adhesion regions formed on the substrate, and cell non-adhesion regions that surround each of the cell adhesion regions;
   (b) a step of seeding a pluripotent stem cell on the cell culture substrate; and
   (c) a step of culturing, in a medium, the pluripotent stem cell seeded.
[3] The method according to [1] or [2], further including (D) a step of proliferating the fibroblast-like cell isolated.
[4] The method according to [1] or [2], wherein, in the step (B), the intestinal structure is cultured in a culture container, and at least part of the intestinal structure is in contact with the culture container.
[5] The method according to [2], wherein a culture period in the step (c) is 60 days or more.
[6] A feeder cell produced using the method according to [1] or [2].
[7] A fibroblast-like cell which is positive for PDGFRA and CD81, and expresses Foxl1 and GREM1.
[8] The fibroblast-like cell according to [7], which is positive for PDGFRA and CD81, is negative for CD34, and expresses Foxl1 and GREM1.
[9] The fibroblast-like cell according to [7], which is derived from a culture product of an intestinal structure that is derived from a pluripotent stem cell or produced from a pluripotent stem cell, and contains an endoderm-derived cell and a mesoderm-derived cell.
[10] The fibroblast-like cell according to [9], wherein the intestinal structure is produced using a method including:
   (a) a step of preparing a cell culture substrate including: a substrate; and a plurality of isolated cell adhesion regions formed on the substrate, and cell non-adhesion regions that surround each of the cell adhesion regions;
   (b) a step of seeding a pluripotent stem cell on the cell culture substrate; and
   (c) a step of culturing, in a medium, the pluripotent stem cell seeded.
[11] The fibroblast-like cell according to [9], wherein the pluripotent stem cell is human-derived.
[12] The feeder cell according to [6] or the fibroblast-like cell according to [7], which is a feeder cell for maintaining or proliferating at least one cell selected from the group consisting of a small-intestine epithelial cell, large-intestine epithelial cell, hepatocyte, and chondrocyte.
[13] The feeder cell according to [6] or the fibroblast-like cell according to [7], which is a feeder cell for maintaining or proliferating the at least one cell by a two-dimensional culture.
[14] The feeder cell according to [6] or the fibroblast-like cell according to [7], which is a feeder cell capable of subculturing the at least one cell three times or more.
[15] A cell sheet including the feeder cell according to [6] or the fibroblast-like cell according to [7].
[16] A coculture product including: at least one cell selected from the group consisting of a small-intestine epithelial cell and a hepatocyte; and the feeder cell according to [6] or the fibroblast-like cell according to [7].
[17] The coculture product according to [16], the passage number of the at least one cell is 3 or more.
[18] A cell sheet including the coculture product according to [16].
[19] The cell sheet according to [18], having a circular or generally circular shape having a diameter of 1 cm or more.
[20] The cell sheet according to [18], including a layer of at least one cell selected from the group consisting of a small-intestine epithelial cell and a hepatocyte.
[21] The cell sheet according to [20], further containing a layer of the feeder cell.
[22] An implant containing the cell sheet according to [15] or [18].
[23] A method of maintaining or proliferating a cell, including a step of culturing the cell in the presence of the feeder cell according to [6] or the fibroblast-like cell according to [7].
[24] The method of maintaining or proliferating a cell according to [23], wherein a culture for the cell is a two-dimensional culture.
[25] The method of maintaining or proliferating a cell according to [23] or [24], wherein the cell is at least one cell selected from the group consisting of a small-intestine epithelial cell, large-intestine epithelial cell, hepatocyte, and chondrocyte.
[26] The method of maintaining or proliferating a cell according to [23] or [24], wherein the cell and the feeder cell are derived from the same individual.
[27] The method of maintaining or proliferating a cell according to [23] or [24], wherein the cell and the feeder cell are derived from different individuals.
[28] A cell which expresses at least one gene selected from the group consisting of DCN, ACTA2, and NOG, and expresses the genes, ADAMTS19, ANO1, BLID, LOC100507053, LHX8, SFRP4, AHRR, SLC14A1, FMO3, PZP, ABCG4, EYA2, TMEM92-AS1, ANO10, EGFL6, SNCAIP, LGSN, PCDHB15, and KRTAP1-5.

### Advantageous Effects of Invention

The present disclosure can provide: a feeder cell for maintaining or proliferating, by a two-dimensional culture, a cell deemed to be difficult to maintain and proliferate by a two-dimensional culture, examples of which cell include an intestinal epithelial cell; and a method of producing the feeder cell. Furthermore, the present disclosure can provide a cell sheet containing such a feeder cell as described above. Furthermore, the feeder cell has a good feeder capability even if subcultured 10 times or more, and thus, can be widely spread without being immortalized. A culture system prepared using the feeder cell can be expected to play a role as a standard model of a culture system closer to a human organism than a mouse embryonic fibroblast (MEF) and an immortalized feeder cell that are feeder cells commonly used. In addition, the feeder cell is adhesive to the surface of a common culture, exhibits the expression of CD90, exists in a positional relationship in which a mesenchymal stem cell originally exists in an organoid, thus can be easily presumed to be one kind of mesenchymal stem cell, and makes it possible to expect a therapeutic effect by transplantation of the feeder cell.

### Brief Description of Drawings

[FIG. 1] FIG. 1 is a photograph of an intestinal structure immunostained using antibodies (an anti-Villin antibody and an anti-5TH antibody) serving as differentiation markers for an intestine, in which the intestinal structure was produced by inducing a pluripotent stem cell to differentiate.
[FIG. 2] FIG. 2 illustrates photographs of a coculture product immunostained using an antibody against markers (CDX2 and Villin) for an intestinal epithelial cell, in which the coculture product was composed of a fibroblast-like cell and an intestinal epithelial cell-like cell that were derived from an intestinal structure.
[FIG. 3] FIG. 3 illustrates photographs of a culture product immunostained using an antibody against a marker (CDX2) for an intestinal epithelial cell, in which the culture product was composed of an intestinal epithelial cell-like cell derived from an intestinal structure.
[FIG. 4] FIG. 4 is a photograph of a coculture product immunostained using an antibody against a marker (CDX2) for an intestinal epithelial cell, in which the coculture product was composed of a fibroblast-like cell and an intestinal epithelial cell-like cell that were derived from an intestinal structure.
[FIG. 5] FIG. 5 illustrates photographs of a coculture product immunostained using an antibody against a marker (Villin) for an intestinal epithelial cell, in which the coculture product was composed of a fibroblast-like cell and an intestinal epithelial cell-like cell that were derived from an intestinal structure.
[FIG. 6] FIG. 6 is a photomicrograph of a coculture product having a passage number of 2, and containing: a fibroblast-like cell derived from an intestinal structure; and a human primary intestinal epithelial cell; in which the photomicrograph was taken at the point of time of culture day 5.
[FIG. 7] FIG. 7 is a photomicrograph of a coculture product having a passage number of 4, and containing: a fibroblast-like cell derived from an intestinal structure; and a human primary intestinal epithelial cell; in which the photomicrograph was taken at the point of time of culture day 4.
[FIG. 8] FIG. 8 is a photomicrograph of a coculture product having a passage number of 2, and containing a mouse embryonic fibroblast (MEF) and a human primary intestinal epithelial cell, in which the photomicrograph was taken at the point of time of culture day 5.
[FIG. 9] FIG. 9 is a photomicrograph of a coculture product having a passage number of 4, and containing a mouse embryonic fibroblast (MEF) and a human primary intestinal epithelial cell, in which the photomicrograph was taken at the point of time of culture day 4.
[FIG. 10] FIG. 10 is a photomicrograph of a coculture product containing: a fibroblast-like cell derived from an intestinal structure; and a hepatocyte; in which the photomicrograph was taken on the 8th day after the start of culture.
[FIG. 11] FIG. 11 illustrates the results obtained by analyzing the gene expression of an intestinal tract and a site-specific cell constituting an intestinal tract, in a coculture product of a fibroblast-like cell and an intestinal epithelial cell-like cell that were derived from an intestinal structure.
[FIG. 12-1] FIG. 12-1 illustrates the results obtained by analyzing the gene expression of an intestinal tract and a site-specific cell constituting an intestinal tract, in a coculture product of a fibroblast-like cell and an intestinal epithelial cell-like cell that were derived from an intestinal structure.
[FIG. 12-2] FIG. 12-2 illustrates the results obtained by analyzing the gene expression of a WNT family of various signaling proteins in a fibroblast-like cell derived from an intestinal structure.
[FIG. 13] FIG. 13 illustrates the results obtained by hierarchical clustering by a comprehensive gene analysis of a coculture product of a fibroblast-like cell and an intestinal epithelial cell-like cell that were derived from an intestinal structure.
[FIG. 14] FIG. 14 illustrates the measurement results of the barrier properties (a trans-epithelial electrical resistance value) of a cell sheet that was a coculture product of a fibroblast-like cell and an intestinal epithelial cell-like cell that were derived from an intestinal structure.
[FIG. 15] FIG. 15 illustrates photographs related to the expression of a transporter of an intestinal epithelial cell in a coculture product of a fibroblast-like cell and an intestinal epithelial cell-like cell that were derived from an intestinal structure.
[FIG. 16] FIG. 16 illustrates the results obtained by analyzing the gene expression of a transporter of an intestinal epithelial cell and a metabolic enzyme in a coculture product of a fibroblast-like cell and an intestinal epithelial cell-like cell that were derived from an intestinal structure.
[FIG. 17] FIG. 17 is a photograph of a coculture product immunostained using an antibody against a marker (Ki-67) of cell proliferation, in which the coculture product was composed of a fibroblast-like cell and an intestinal epithelial cell-like cell that were derived from an intestinal structure.
[FIG. 18] FIG. 18 is photographs of a coculture product stained using Alcian blue, in which the coculture product was composed of a fibroblast-like cell and an intestinal epithelial cell-like cell that were derived from an intestinal structure.
[FIG. 19] FIG. 19 is a graph illustrating the feeder capability at each passage number of a fibroblast-like cell derived from an intestinal structure.
[FIG. 20] FIG. 20 is a photograph of a coculture product immunostained using an antibody against each of the markers (CDX2 and Villin) for an intestinal epithelial cell, in which the coculture product was composed of a conventional feeder cell and an intestinal epithelial cell-like cell.
[FIG. 21] FIG. 21 illustrates graphs illustrating the expression of lubricin and COL2A1 in each of a fibroblast-like cell derived from an intestinal structure and a chondrocyte collected from a human child.
[FIG. 22] FIG. 22 illustrates photographs of a fibroblast-like cell immunostained using an antibody against each of PDGFRA and CD81, in which the fibroblast-like cell was derived from an intestinal structure.
[FIG. 23] FIG. 23 illustrates the results obtained by analyzing the gene expression of each of Foxl1, CD34, and GREM1 in a fibroblast-like cell derived from an intestinal structure.
[FIG. 24] FIG. 24 is a photograph of a cell sheet produced using a fibroblast-like cell derived from an intestinal structure.

### Description of Embodiments

As used herein, the term "feeder cell" refers to a cell to be cultured (cocultured) together with a cell of interest, a cell before differentiation into the cell of interest, or the like in order to prepare suitable culture conditions for promoting the maintenance and/or proliferation of the cell of interest, in which the cell to be cultured is different from the cell of interest. In the present disclosure, the feeder cell may be seeded at the same time as the cell of interest, or may be seeded before the cell of interest is seeded. In one embodiment, a feeder cell is seeded before the cell of interest is seeded, and thus forms a layer of a feeder cell (i.e., a feeder layer). The cell of interest and the feeder cell may be cultured in contact with each other, or may be cultured without contact with each other. In one embodiment, a cell of interest and a feeder cell are cultured in such a manner that at least part of the cell of interest is in contact with the feeder cell. In this regard, the proliferation of a feeder cell may be inhibited using a known method, such as irradiation (for example, with γ rays) or treatment with an antibiotic (for example, mitomycin C).

As used herein, the term "pluripotent stem cell" refers to a cell that has what is called pluripotency for differentiation. As used herein, the terms "pluripotency" and "pluripotency for differentiation" refer to the capability of a cell to differentiate into all germ layers (i.e., ectoderm, mesoderm, and endoderm) included in an organism. Examples of the pluripotent stem cell include an embryonic stem cell (ES cell), ES cell derived from a somatic cell (ntES cell), induced pluripotent stem cell (iPS cell), adult tissue, and stem cell derived from umbilical cord blood or an umbilical cord.

As used herein, the term "fibroblast-like cell" refers to not only what is called a fibroblast but also a cell resulting from differentiation of a fibroblast, and having an oblate or spindle-like shape, examples of which cell include a myofibroblast. In addition, the origin of the fibroblast-like cell is not particularly limited, and the fibroblast-like cell may be, for example, a cell derived from the above-described pluripotent stem cell or the below-described intestinal structure. In addition, a fibroblast-like cell can be a cell that exhibits the below-described specific gene expression profile. In this regard, a "fibroblast-like cell" in the present disclosure does not encompass the above-described various cells that are naturally-occurring, but refers to a cell obtained by artificially manipulating a naturally-occurring cell or an artificially produced cell, i.e., refers to an artificially produced cell (artificial cell).

### [Method of producing feeder cell]

One aspect of the present disclosure provides a method of producing a feeder cell for maintaining a cell deemed to be difficult to maintain and proliferate by a two-dimensional culture, examples of which cell include an intestinal epithelial cell (the feeder cell is hereinafter also referred to as a "feeder cell according to the present disclosure"). A feeder cell according to the present disclosure can be produced using a method including the following steps (A) to (C) (the method is hereinafter also referred to as a "producing method according to the present disclosure"):
(A) a step of preparing an intestinal structure produced from a pluripotent stem cell, and containing an endoderm-derived cell and a mesoderm-derived cell;
(B) a step of culturing the intestinal structure to proliferate a fibroblast-like cell derived from the intestinal structure, and
(C) a step of isolating the fibroblast-like cell to obtain a feeder cell. Each of the steps (A) to (C) will be described in detail below.

### <Step (A)>

In step (A), an intestinal structure produced from a pluripotent stem cell, and containing an endoderm-derived cell and a mesoderm-derived cell is prepared. The intestinal structure is a structure containing an endoderm-derived cell and a mesoderm-derived cell, and having at least one function the same as or similar to a function of an intestine.

The intestinal structure is not particularly limited, subject to having the above-described cellular constitution and functions, and can be obtained, for example, by inducing a pluripotent stem cell to differentiate. In one embodiment, an intestinal structure used in a producing method according to the present disclosure can be produced using a method including the following steps (a) to (c).

### (Step (a))

Step (a) is a step of preparing a cell culture substrate including: a substrate; and a plurality of isolated cell adhesion regions (regions having cell-adhesiveness) formed on the substrate, and cell non-adhesion regions (regions having cell-nonadhesiveness) that surround each of the cell adhesion regions.

As used herein, the term "cell-adhesiveness" means the strength of adhesion of a cell, i.e., the easiness of adhesion of a cell. Accordingly, the "cell adhesion region" means a region having relatively high cell-adhesiveness, and the "cell non-adhesion region" means a region having relatively low cell-adhesiveness. Specifically, cell-adhesiveness means a degree to which adhesion or extension of a cell occurs depending on the chemical property, physical property, and the like of a substrate (the surface of the substrate). Cell adhesion occurs more easily in a cell adhesion region having relatively high cell-adhesiveness, and cell adhesion occurs less easily in a cell adhesion region having relatively low cell-adhesiveness. Accordingly, in a case where, as described above, cells are seeded on a substrate in which a cell adhesion region and a cell non-adhesion region form a pattern, the cells adhere more easily to the cell adhesion region, the cells adhere less easily to the cell non-adhesion region, and thus, the cells seeded are arranged in patterned form on a substrate.

The cell-adhesiveness can be judged using an extensional ratio of cell adhesion as an index, in which the extensional ratio is determined by culturing cells seeded on a substrate, and arranged in pattern form. The higher the extensional ratio of cell adhesion, the more efficient the cells can be cultured. The extensional ratios of cell adhesion of the cell adhesion region and the cell non-adhesion region are not particularly limited, subject to achieving the effects of the present disclosure, and can be, for example, 60% or more and less than 60% respectively. The extensional ratio of cell adhesion of the cell adhesion region is preferably 70% or more, more preferably 80% or more, still more preferably 90% or more. On the other hand, the extensional ratio of cell adhesion of the cell non-adhesion region is preferably less than 40%, more preferably 5% or less, still more preferably 2% or less. The extensional ratio of cell adhesion is defined as ({(number of cells adhering to surface of measuring object) / (number of cells seeded on surface of measuring object)} ×100 (%)) that is the ratio of the cells adhering and extended at the point of time when cells to be cultured are seeded on the surface of a measuring object in the seeding-density range of from 4,000 cells/cm² or more and less than 30,000 cells/cm², stored in an incubator at a temperature of 37°C and at a CO₂ concentration of 5%, and cultured for 14.5 hours.

The cells are seeded on the measuring object, as follows; the cells are suspended in a Dulbecco's modified Eagle's medium (DMEM) supplemented with FBS at 10%; the resulting cell suspension is added to the measuring object; and then, the measuring object having the cells added thereto are shaken slowly, so that the cells are distributed as uniformly as possible. Furthermore, the extensional ratio of cell adhesion is measured after the cells not adhering to the measuring object are removed by exchanging the medium immediately before the measurement. In the measurement of the extensional ratio of cell adhesion, the measurement portion is the portion excluding the portion where the density of presence of cells tends more to be specific (for example, the center of a predetermined region in which center the density of presence tends more to be high, and the outer edge of the predetermined region in which outer edge the density of presence tends more to be low).

In the present disclosure, a substrate to be used to produce an intestinal structure is not particularly limited, subject to achieving the effects of the present disclosure, and is, for example, a substrate in which a cell non-adhesion region is formed by fixing polyethylene glycol on the substrate, and in which a cell adhesion region is formed by performing an oxidizing treatment and/or a decomposing treatment on at least part of the polyethylene glycol fixed on the substrate. Such a substrate can be produced, for example, as follows: a thin film of polyethylene glycol (PEG) is formed on the whole surface of a substrate; and then, a region to which cells are desired to adhere undergoes an oxidizing treatment and/or a decomposing treatment to be provided with cell-adhesiveness, whereby a cell adhesion region is formed. A portion that has undergone neither an oxidizing treatment nor a decomposing treatment becomes a cell non-adhesion region having PEG fixed therein.

The substrate is not particularly limited, subject to being formed with a material on the surface of which a thin PEG film can be formed. Examples of a material for the substrate include: inorganic materials, such as metal, glass, ceramic, and silicon; and organic materials typified by elastomers and plastics (for example, a polystyrene resin, polyester resin, polyethylene resin, polypropylene resin, ABS resin, nylon, acryl resin, fluorine resin, polycarbonate resin, polyurethane resin, methylpentene resin, phenol resin, melamine resin, epoxy resin, and vinyl chloride resin). The shape of the substrate is not particularly limited. Examples include: flat shapes, such as a flat plate, flat film, film, and porous film; and solid shapes, such as a cylinder, stump, multi-well plate, and micro flow path. In one embodiment, a film-shaped substrate is used. The thickness of the film-shaped substrate is not particularly limited, and is usually 0.1 to 1,000 µm, preferably 1 to 500 µm, more preferably 10 to 200 µm.

Polyethylene glycol (PEG) contains at least an ethylene glycol chain (EG chain) composed of one or more ethylene glycol units (-(CH₂)₂-O-). The polyethylene glycol chain may be linear or branched. The ethylene glycol chain refers to a structure, for example, represented by the following formula:

-((CH₂)₂-O)ₘ-

(m is an integer that represents a degree of polymerization).
m is not particularly limited, subject to achieving the effects of the present disclosure, and is preferably 1 to 13, more preferably 1 to 10.

The PEG encompasses an ethylene glycol oligomer. In addition, the PEG encompasses a PEG having a functional group introduced thereinto. Examples of the functional group include an epoxy group, carboxyl group, N-hydroxysuccinimide group, carbodiimide group, amino group, glutaraldehyde group, and (meth)acryloyl group. The functional group is introduced preferably into the end of a PEG chain, via a linker, if desired. Examples of the PEG having the functional group introduced thereinto include PEG (meth)acrylate and PEG di(meth)acrylate.

The average thickness of a thin PEG film to be formed on the substrate is not particularly limited, and is preferably 0.8 nm to 500 µm, more preferably 0.8 nm to 100 µm, still more preferably 1 nm to 10 µm, particularly preferably 1.5 nm to 1 µm. A thin PEG film having an average thickness of 0.8 nm or more makes it more unlikely that the adsorption of a protein and the adhesion of a cell are influenced by a region that is on the surface of the substrate, and not covered with the thin PEG film. On the other hand, the average thickness of 500 µm or less makes it possible to perform coating relatively easily. Furthermore, allowing the thin PEG film to have a given or larger thickness allows the cell-nonadhesiveness to decrease, thus enabling a cell to be inhibited from adhering or extending to a region other than the cell adhesion region. In addition, allowing the thin PEG film to have a given or smaller thickness makes it possible that a factor necessary for cell survival, and contained in a culture liquid is also spread to a cell close to the substrate in the cell adhesion region.

Examples of a method of forming a thin PEG film on a substrate include: a method in which PEG is directly adsorbed into the surface of a substrate; a method in which the surface of a substrate is directly coated with PEG; a method in which the surface of a substrate is coated with PEG, which then undergoes a cross-linking treatment; a method in which an underlying layer is formed on the surface of a substrate to increase adhesiveness between the substrate and a thin PEG film, and then coated with PEG; and a method in which the starting point for polymerization is formed on the surface of a substrate, and then, PEG is polymerized. In a preferable embodiment, a thin PEG film is formed on a substrate by forming an underlying layer on the substrate, and then coating the underlying layer with PEG.

The underlying layer on the surface of the substrate can be formed, for example, using a method described in JP2012-175983A. In a preferable embodiment, the underlying layer on the surface of the substrate can be formed by fixing a silane coupling agent on the surface of the substrate, in which the silane coupling agent has a functional group capable of forming a covalent bond through reaction with a hydroxyl group at a PEG end or with a functional group introduced, or has a functional group capable of being converted to such a functional group. Examples of such a functional group include a (meth)acryloyl group, (1H-imidazole-1-yl)carbonyl group, succinimidyloxycarbonyl group, glycidyl group, epoxy group, aldehyde group, amino group, thiol group, carboxyl group, azido group, cyano group, active ester group (1H-benzotriazole-1-yloxycarbonyl group, pentafluorophenyloxycarbonyl group, paranitrophenyloxycarbonyl group, and the like), carbonyl halide group, isocyanate group, and maleimide group. Among these functional groups, preferable functional groups are a (meth)acryloyl group, glycidyl group, and epoxy group.

For example, in a case where a silane coupling agent (methacryloyl silane) having a methacryloyl group at an end is used, the water contact angle of the surface of the substrate having a methacryloyl silane added thereto is typically 45° or more, preferably 47° or more, more preferably 48° or more, still more preferably 50° or more. In this regard, a water contact angle in the present disclosure refers to a water contact angle measured at 23°C. In a case where the surface of the substrate has such a water contact angle as described above, PEG can be fixed at a sufficient density.

The density and cell-adhesiveness of PEG fixed on the substrate can be simply evaluated using the water contact angle of the surface of the substrate as an index. Specifically, the lower the water contact angle of the surface of the substrate, the higher the density of PEG present on the surface of the substrate. As a result, a region having low cell-adhesiveness (i.e., a cell non-adhesion region) is formed. That is, for example, in a case where the water contact angle of the surface of the substrate is, for example, 48° or less, preferably 40° or less, more preferably 30° or less, after PEG is fixed on the surface of the substrate, it is considered that PEG having a sufficient density is present on the surface of the substrate, and that the cell-adhesiveness is sufficiently low.

In the present disclosure, an oxidizing treatment to be performed on polyethylene glycol to form a cell adhesion region refers to a reaction in which an organic compound, i.e., PEG reacts with oxygen to contain oxygen in a larger amount than before the reaction. In addition, in the present disclosure, a decomposing treatment to be performed on polyethylene glycol to form a cell adhesion region refers to a reaction in which a bond of an organic compound, i.e., PEG, is cleaved. Examples of the "decomposing treatment" typically include, but are not limited to, decomposition by oxidation and decomposition by ultraviolet irradiation. In this regard, in a case where the "decomposing treatment" is decomposition caused by oxidation (i.e., oxidative decomposition), the "decomposing treatment" and the "oxidizing treatment" refer to the same treatment.

The decomposition by ultraviolet irradiation means that PEG absorbs ultraviolet rays, undergoes an excited state, and is decomposed. In this regard, in a case where ultraviolet rays are radiated to a system in which PEG and a molecular species containing oxygen (for example, oxygen or water) coexist, the PEG absorbs the ultraviolet rays, and is decomposed, and besides, the molecular species containing oxygen is activated and reacts with the PEG in some cases. In this case, the latter reaction is classified into "oxidation". In addition, the reaction in which PEG is decomposed by oxidation due to the molecular species activated is classified not into "decomposition by ultraviolet irradiation" but into "decomposition by oxidation". In such a manner, "oxidation" and "decomposition" as operations overlap each other in some cases, and cannot be clearly distinguished in some cases. Herein, such a case is also expressed as "oxidation and/or decomposition".

Examples of a method of oxidation and/or decomposition include a method in which a thin PEG film is treated with ultraviolet irradiation; a method in which a thin PEG film is treated with a photocatalyst; and a method in which a thin PEG film is treated with an oxidizing agent. Oxidation and/or decomposition of part of a thin PEG film can be performed using a mask, such as a photomask or a stencil mask, or a stamp. In addition, oxidation and/or decomposition can be performed using a direct-writing method, such as a method to be performed using a laser, such as an ultraviolet laser.

In an ultraviolet irradiation treatment, a light source to be used is preferably a lamp that radiates ultraviolet rays between the VUV region and the UV-C region, such as a mercury lamp that radiates ultraviolet rays at a wavelength of 185 nm or 254 nm or an excimer lamp that radiates ultraviolet rays at a wavelength of 172 nm. In a photocatalyst treatment, a light source that radiates ultraviolet rays at a wavelength of 365 nm or less is preferably used, and a light source that radiates ultraviolet rays at a wavelength of 254 nm or less is more preferably used. Examples of a photocatalyst to be preferably used include a titanium oxide photocatalyst and a titanium oxide photocatalyst activated with a metal ion or metal colloid. In addition, as an oxidizing agent, an organic acid or an inorganic acid can be used without particular limitation, but a high-concentration acid is difficult to handle, and thus, is preferably diluted to a concentration of 10% or less to be used. An optimal ultraviolet-ray treatment time, photocatalyst treatment time, and oxidizing-agent treatment time can be suitably determined depending on the conditions, such as the ultraviolet-ray intensity of a light source to be used, the activity of a photocatalyst, and the oxidative power and concentration of an oxidizing agent.

In one embodiment, the carbon content of a cell adhesion region (including an underlying layer in a case where the underlying layer exists) is preferably lower than the carbon content of a cell non-adhesion region (including an underlying layer in a case where the underlying layer exists). Specifically, the carbon content of a cell adhesion region is preferably 20 to 99% of the carbon content of a cell non-adhesion region. In addition, the value of the ratio (%) of oxygen-bound carbon in the carbon in a cell adhesion region (including an underlying layer in a case where the underlying layer exists) is preferably a value smaller than the value of the ratio (%) of oxygen-bound carbon in the carbon in a cell non-adhesion region (including an underlying layer in a case where the underlying layer exists). Specifically, the value of the ratio (%) of oxygen-bound carbon in the carbon in a cell adhesion region is preferably 35 to 99% of the value of the ratio (%) of oxygen-bound carbon in the carbon in a cell non-adhesion region. In a case where cells seeded are arranged in pattern form on a substrate, the cell-adhesiveness increases as the ultraviolet-ray exposure increases. This is because, if the adhesiveness is high, the cells tend less to be detached during collection of the cells, thus becoming difficult to collect.

As used herein, the "carbon content" of a cell adhesion region is defined as the "carbon content determined from the analytical value of a C1s peak obtained using an X-ray photoelectron spectrometer", and the "ratio of oxygen-bound carbon" is defined as the "ratio of oxygen-bound carbon determined from the analytical value of a C1s peak obtained using an X-ray photoelectron spectrometer".

In a substrate to be used in the present disclosure, each cell adhesion region is patterned so as to have an area in the range of larger than 0.785 mm², preferably 1.0 mm² or more, more preferably 1.2 mm² or more, still more preferably 1.5 mm² or more, most preferably 1.7 mm² or more, and preferably 25 mm² or less, more preferably 15 mm² or less, still more preferably 10 mm² or less, most preferably 5 mm² or less. Culturing a pluripotent stem cell allowed to adhere to a cell adhesion region having an area larger than 0.785 mm² makes it possible to culture the cell in an adhering state without allowing the cell to be detached, and thus, promotes induction of differentiation into an intestinal structure. In addition, culturing a pluripotent stem cell allowed to adhere to a cell adhesion region having an area of 25 mm² or less enables differentiation into an intestinal structure to be induced effectively.

The shape of each cell adhesion region is not particularly limited, and can be, for example, a polygon, typically a quadrangle, or a circle or an ellipse. Among these, a circle is preferable. A circle having a diameter of more than 1.0 mm, preferably 1.2 mm or more, more preferably 1.5 mm or more, and preferably 6 mm or less, more preferably 4 mm or less, still more preferably 3 mm or less, particularly preferably 2 mm or less, is preferable. In one substrate, a plurality of cell adhesion regions may be the same as or different from each other in area and/or shape, and are preferably the same in area and shape.

In addition, in a substrate, the cell adhesion regions are each surrounded by a cell non-adhesion region, i.e., are isolated from each other, preferably disposed 0.75 mm or more, more preferably 1.5 mm or more, apart from each other. That is, the cell adhesion regions are disposed in such a manner that the shortest distance therebetween (in the case of a circle, the distance between the centers of two circles is a value obtained by adding the above-described value to the total of the radii) is preferably 0.75 mm or more, more preferably 1.5 mm or more. Isolating the cell adhesion regions at a given or longer distance from each other allows the cells in each cell adhesion region to be cultured uniformly at given intervals without forming an intercellular junction with a cell in another cell adhesion region, and thus makes it possible to construct an experiment system with high reproducibility.

The ratio of the cell adhesion regions in a substrate is usually 5 to 80%, preferably 20 to 70%, more preferably 40 to 60%. In this regard, even in a case where the substrate is placed in a dish (Schale) or the like, this ratio is the ratio of the cell adhesion regions to the whole substrate, not taking the bottom face of the dish into consideration. Having cells in a given or larger amount with respect to a culture liquid makes it possible to prevent cell death, and having cells in a given or smaller amount makes it possible to prevent depletion of a factor necessary for survival, and prevent the cells from being damaged by the depletion.

In addition, the cell adhesion regions are preferably disposed regularly at given intervals, for example, in the form of a grid having the same pitch lengthwise and crosswise. Having a constant paracrine effect due to a substance produced from a cell in each cell adhesion region enables an influence on differentiation to be constant.

For example, a pattern having a plurality of circular cell adhesion regions can be formed as follows: a photomask having a plurality of circular openings is used; a glass base plate having a thin PEG film formed thereon and the photomask are disposed so as to be opposed to each other; the glass base plate is irradiated with ultraviolet rays from the photomask side; and the regions that are in the thin PEG film, and correspond to the openings of the photomask are oxidized.

A substrate to be used in the present disclosure preferably undergoes a precoating treatment for the purpose of promoting adhesion of a pluripotent stem cell to a cell adhesion region. The precoating treatment can be performed by coating the substrate with an extracellular matrix (for example, collagen, fibronectin, proteoglycan, laminin, or vitronectin), gelatin, lysine, peptide, a gelatinous matrix containing any of these, serum, or the like. The precoating treatment of the substrate can promote the adhesion of a less adhesive pluripotent stem cell (for example, an ES cell or iPS cell) to a cell adhesion region, enabling the cell to effectively undergo an adhesion culture and induction of differentiation.

In the same manner, it is preferable that, for the purpose of promoting the adhesion of a pluripotent stem cell to a cell adhesion region, a feeder cell is seeded and cultured approximately 24 hours before the pluripotent stem cell is seeded, and that the pluripotent stem cell is then cultured on the feeder cell. As the feeder cell, a feeder cell that is usually used in the art can be used without particular limitation. Examples include a fibroblast. The feeder cell is seeded at a density of less than 1.26 × 10⁵ cells/cm², preferably a density of 6.3 × 10⁴ cells/cm² or less, preferably a density of 3.15 ×10⁴ cells/cm² or more, with respect to the cell culture substrate.

The above-described precoating treating and the seeding of a feeder cell may each be performed singly, or both may be performed in combination. Preferably, any one of the precoating treatment and the seeding of a feeder cell is performed.

### (Step (b))

The step (b) is a step of seeding a pluripotent stem cell on a cell culture substrate prepared in the above-described step (a), and including a cell adhesion region and a cell non-adhesion region.

Examples of the pluripotent stem cell to be seeded on the cell culture substrate include an embryonic stem cell (ES cell), ES cell derived from a somatic cell (ntES cell), induced pluripotent stem cell (iPS cell), adult tissue, and stem cell derived from umbilical cord blood or an umbilical cord. These pluripotent stem cells may be used singly, or may be used in combination of two or more kinds thereof. In a preferable embodiment, an ES cell and an iPS cell are each used singly as the pluripotent stem cell.

An ES cell to be used in the present disclosure is preferably an ES cell derived from a mammal, and can be an ES cell derived from, for example, a rodent, such as a mouse, or a primate, such as a human. In a preferable embodiment, an ES cell to be used is an ES cell derived from a mouse or a human. An ES cell refers to a stem cell line produced from an inner cell mass belonging to part of an embryo in the blastocyst stage that is the brephic stage of an animal, and can be proliferated in vitro substantially infinitely, keeping pluripotency that allows differentiation into all tissues in theory. Examples of an ES cell that can be used include a cell having a reporter gene introduced into the vicinity of the Pdx1 gene to make it easier to verify the degree of differentiation of the ES cell. For example, it is possible to use a 129/Sv-derived ES cell line having a LacZ gene incorporated into the Pdx1 locus, or an ES cell SK7 line having a GFP reporter transgene under the control of a Pdx1 promoter. Alternatively, it is possible to use an ES cell PH3 line having an mRFP1 reporter transgene under the control of an Hnf3β endoderm-specific enhancer fragment and a GFP reporter transgene under the control of a Pdx1 promoter.

An iPS cell to be used in the present disclosure is a pluripotent cell obtained by reprogramming a somatic cell. An induced pluripotent stem cell has been successfully produced by a plurality of groups including: the group of Prof. Shinya Yamanaka, Kyoto University; the group of Rudolf Jaenisch, Massachusetts Institute of Technology; the group of James Thomson, University of Wisconsin; and the group of Konrad Hochedlinger, Harvard University. For example, WO2007/069666 describes: a nuclear reprogramming factor for a somatic cell, the factor including a gene product of each of an Oct family gene, Klf family gene, and Myc family gene; and a nuclear reprogramming factor for a somatic cell, the factor including a gene product of each of an Oct family gene, Klf family gene, Sox family gene, and Myc family gene; and further describes a method of producing an induced pluripotent stem cell by nuclear reprogramming of a somatic cell, the method including a step of bringing a somatic cell into contact with the nuclear reprogramming factor.

A somatic cell that can be used here is not particularly limited to any kind, and can be an arbitrary somatic cell. That is, a somatic cell herein encompasses all the cells other than a germ cell in the cells constituting an organism, may be a differentiated somatic cell, or may be an undifferentiated stem cell. The origin of the somatic cell may be, but is not particularly limited to, any of mammals, birds, fishes, reptiles, and amphibians, and is preferably a mammal (for example, a rodent, such as a mouse, or a primate, such as a human), particularly preferably a mouse or a human. In addition, in a case where a somatic cell of a human is used, a somatic cell of any of an embryo, a newborn, or an adult can be used. Examples of the somatic cell include a fibroblast (for example, dermal fibroblast), epithelial cell (for example, gastric epithelial cell, liver epithelial cell, and alveolar epithelial cell), endothelial cell (for example, blood vessel and lymph vessel), neurocyte (for example, neuron and gliacyte), pancreatic cell, blood cell, bone-marrow cell, muscle cell (for example, skeletal muscle cell, smooth muscle cell, and myocardial cell), hepatic parenchymal cell, non-hepatic parenchymal cell, adipocyte, osteoblast, cell constituting a periodontal tissue (for example, periodontal ligament cell, cementoblast, gingival fibroblast, and osteoblast), and cell constituting a kidney, eye, or ear.

An iPS cell refers to a stem cell having a replication competence under predetermined culture conditions (for example, conditions for culturing an ES cell) over a long period of time, and in addition, having pluripotency for an ectoderm, mesoderm, and endoderm under predetermined differentiation-inducing conditions. In addition, an iPS cell in the present disclosure may be a stem cell having the capability to form a teratoma when transplanted in a test animal, such as a mouse.

To produce an iPS cell from a somatic cell, at least one kind of reprogramming gene is first introduced into a somatic cell. A reprogramming gene is a gene encoding a reprogramming factor having the action of reprogramming a somatic cell to convert the somatic cell to an iPS cell. Specific examples of a combination of reprogramming genes include, but are not limited to, the following combinations:
(i) Oct gene, Klf gene, Sox gene, and Myc gene;
(ii) Oct gene, Sox gene, NANOG gene, and LIN28 gene;
(iii) Oct gene, Klf gene, Sox gene, Myc gene, hTERT gene, and SV40 large T gene; and
(iv) Oct gene, Klf gene, and Sox gene.

As a pluripotent stem cell to be used before being seeded in a cell culture substrate is a pluripotent stem cell that maintains undifferentiatedness using a medium that does not induce differentiation. Before and after being seeded on the surface of the cell culture substrate, the medium is exchanged with a differentiation-inducing medium, and the cell is seeded on the surface of the cell culture substrate. The cell is directly proliferated until the cells are confluent in a cell adhesion region. The resulting cell aggregate is treated with an enzyme, enabling a differentiation-induced cell of interest to be obtained.

A medium that does not induce differentiation, and is to be used in the present disclosure is not particularly limited, subject to being a medium that does not induce a pluripotent stem cell to differentiate. Examples include: a medium containing a leukemia inhibitory factor known as having the property of maintaining the undifferentiatedness of a mouse ES cell and a mouse iPS cell; a medium containing a basic FGF known as having the property of maintaining the undifferentiatedness of a human iPS cell. The differentiation-inducing medium is not particularly limited, subject to being a medium that induces a pluripotent stem cell to differentiate. Examples include a serum-containing medium and a serum-free media containing a known component having the property of substituting for serum. Depending on the kind of a cell to be used, an MEM medium, BME medium, DMEM medium, DMEM-F12 medium, aMEM medium, IMDM medium, ES medium, DM-160 medium, Fisher medium, F12 medium, WE medium, RPMI1640 medium, or the like can be used.

To the above-described various media, various growth factors, antibiotics, amino acids, and the like may be added. For example, 0.1 to 2% pyruvic acid, 0.1 to 2% non-essential amino acid, 0.1 to 2% penicillin/streptomycin, 0.1 to 1% glutamine, 0.1 to 2% β mercaptoethanol, or 1 to 20 mM ROCK inhibitor (for example, Y27632) may be added. To the differentiation-inducing medium, a humoral factor may be added, but a method of inducing differentiation into an intestinal structure in the present disclosure can induce differentiation into the intestinal structure without the addition of a humoral factor. Accordingly, in one embodiment, a cell is cultured using a differentiation-inducing medium containing no humoral factor.

A method of seeding a pluripotent stem cell on a cell culture substrate is not particularly limited. A cell can be seeded using a method that is usually used in a case where the cell is seeded. In the present disclosure, a pluripotent stem cell is seeded, for example, at a density of less than 1.2 × 10⁵ cells/cm², preferably 3 × 10⁴ cells/cm² or less, preferably 1.5 ×10⁴ cells/cm² or more, with respect to the cell culture substrate.

### (Step (c))

The step (c) is a step in which a pluripotent stem cell seeded on a cell culture substrate in the above-described step (b) is cultured in a medium to obtain an intestinal structure.

A pluripotent stem cell is cultured usually at 37°C. The culture is preferably performed in an atmosphere having a CO₂ concentration of approximately 5%, using a CO₂ cell culture device or the like.

A medium to be used in the present step (c) is not particularly limited, subject to being a medium for inducing a pluripotent stem cell to differentiate, and can be suitably selected in accordance with the kind of the pluripotent stem cell. Examples of the medium include an MEM medium, BME medium, DMEM medium, DMEM-F12 medium, aMEM medium, IMDM medium, ES medium, DM-160 medium, Fisher medium, F12 medium, WE medium, and RPMI1640 medium. In addition, to these media, serum or the property of substituting for serum may be added, if desired.

The culture period after seeding of a pluripotent stem cell on a cell culture substrate is preferably 60 days or more, more preferably 80 days or more, still more preferably 100 days or more, and preferably 210 days or less, more preferably 140 days or less. It has been discovered that, on the 60th day after a pluripotent stem cell is seeded on a cell culture substrate, Villin and 5TH, differentiation markers for an intestine, are detected, furthermore a saccate structure is verified, and a peristaltic-movement-like movement is observed. An intestinal structure obtained by the steps (a) to (c) contains at least an endoderm-derived cell and a mesoderm-derived cell, and may contain another cell.

As described above, the steps (a) to (c) can afford an intestinal structure produced from a pluripotent stem cell, and containing an endoderm-derived cell and a mesoderm-derived cell. The resulting intestinal structure is used in the following step (B).

### <Step (B)>

In the step (B), the intestinal structure prepared in the above-described step (A) is cultured, whereby a fibroblast-like cell derived from the intestinal structure is proliferated.

The form of a culture of an intestinal structure is not particularly limited, subject to achieving the effects of the present disclosure, may be a form in which a cell is allowed to adhere to a culture container, and cultured (what is called an adhesion culture), or may be a form in which a cell is allowed to adhere to a culture container, and cultured (what is called a suspension culture). In a preferable embodiment, an intestinal structure is cultured in a state where at least part of the intestinal structure is in contact with a culture container. The culture container and the intestinal structure may constantly in contact with each other, or may be intermittently in contact with each other.

In one embodiment, an intestinal structure is cultured in a suspension culture in a culture container, and the amount of a medium added to the culture container is suitably regulated, whereby the culture container (for example, the bottom face or the inner wall) is brought into contact with at least part of the intestinal structure. For example, in a case where a dish is used as a culture container, a medium is added shallowly to the dish, and a suspension culture is performed in such a manner that at least part of the intestinal structure is in contact with the bottom face of the dish.

A medium to be used in the present step (B) is not particularly limited, subject to being a medium for inducing differentiation into a fibroblast, and proliferating the fibroblast. Examples of the medium include an MEM medium, BME medium, DMEM medium, DMEM-F12 medium, aMEM medium, IMDM medium, ES medium, DM-160 medium, Fisher medium, F12 medium, WE medium, and RPMI1640 medium. In addition, to these media, serum or the property of substituting for serum may be added, if desired.

An intestinal structure is cultured usually at 37°C. The culture is preferably performed in an atmosphere having a CO₂ concentration of approximately 5%, using a CO₂ cell culture device or the like.

The intestinal structure is preferably cultured until a fibroblast-like cell derived from the intestinal structure is confluent or substantially confluent. The culture period can be suitably set in accordance with the speed of proliferation of the fibroblast-like cell, the shape and size of a culture container to be used, and the like. The culture period of the intestinal structure may be, for example, 1 to 6 days, 6 to 12 days, or 12 to 72 days.

In the present step (B), a cell having an intestinal epithelial cell-like property (the cell is hereinafter also referred to as an "intestinal epithelial cell-like cell") is proliferated in addition to the above-described fibroblast-like cell in some cases. In such a manner, an intestinal epithelial cell-like cell derived from an intestinal structure, and obtained using the present step (B) is different in the property from an intestinal epithelial cell obtained by a conventional organoid culture to be performed using a stem cell as a starting point. For example, the present inventors have demonstrated that, in an intestinal epithelial cell-like cell derived from an intestinal structure, and obtained using the present step (B), a cell division-associated protein Ki-67 that is a proliferative cell marker is present locally. Here, it is common that an intestinal epithelial cell is generated only through differentiation from a stem cell positive for Lgr5 (Leucine-rich repeat-containing G-protein-coupled receptor 5) known as an intestinal epithelial stem cell, and it is commonly known that an intestinal epithelial cell after differentiation is not generated through proliferation. Accordingly, it can be said that the local presence of the proliferative cell marker Ki-67 in an intestinal epithelial cell-like cell derived from an intestinal structure, and obtained using the present step (B) as described above suggests that an intestinal epithelial cell-like cell undergoes division to generate a new intestinal epithelial cell-like cell. That is, it is considered that an intestinal epithelial cell-like cell derived from an intestinal structure, and obtained using the present step (B) has a peculiar property in terms of having the possibility of proliferating without depending on a conventional stem cell positive for Lgr5. In addition, as intestinal epithelial cell-like cells derived from an intestinal structure, and obtained using the present step (B), various kinds of cells that bear the function of a small intestine are obtained, such as a secretion-system cell to be verified by the expression of a specific mucilaginous substance or an absorptive epithelial cell to be verified by the expression of a specific transporter.

A fibroblast-like cell (i.e., a feeder cell according to the present disclosure) derived from an intestinal structure, and obtained using the present step (B) and an intestinal epithelial cell-like cell may be combined with each other, or may each be used singly.

### <Step (C)>

In the step (C), a fibroblast-like cell derived from an intestinal structure, and proliferated in the present step (B), i.e., a feeder cell according to the present disclosure is isolated.

A fibroblast-like cell can be isolated using a method that is usually used to isolate a fibroblast. That is, the isolation can be performed using a step such as a known enzyme treatment or scraping with a scraper. These steps may be used singly, or may be used in combination of two or more kinds thereof.

In one embodiment, a fibroblast-like cell obtained using the present step (C) expresses at least one fibroblast marker selected from the group consisting of vimentin and CD90. In a preferable embodiment, a fibroblast-like cell obtained using the present step (C) expresses at least one gene selected from the group consisting of ACTA2, SOX9, and DCN.

In one embodiment, isolation of an intestinal epithelial cell-like cell derived from an intestinal structure is performed in the step (C), in addition to isolation of the above-described fibroblast-like cell derived from an intestinal structure. Specifically, an intestinal epithelial cell-like cell derived from an intestinal structure can be isolated in accordance with the following procedure. First, a medium which contains an intestinal structure, and from which a fibroblast-like cell has been isolated as described above is made into an environment that enables only an intestinal epithelial cell-like cell to survive. For example, by adding an antibiotic, such as puromycin or penicillin, at 100 µg/ml to the medium, the medium is made into an environment in which only an intestinal epithelial cell-like cell having an activity (drug-metabolizing enzyme: CYP) that metabolizes the antibiotic can survive. Then, a dead cell is removed from the medium, and an intestinal epithelial cell-like cell is isolated. In this regard, in a case where a hepatocyte is present in the medium, adding an antibiotic as described above enables the hepatocyte to survive, but the hepatocyte can be easily distinguished as a population of coenocytes under a microscope, and thus, removing the hepatocyte under a microscope enables an intestinal epithelial cell-like cell to be isolated. In addition, a hepatocyte is not stained by immunostaining performed using an antibody against CDX2, and thus, distinguishing and removing the hepatocyte using such an antibody also enables an intestinal epithelial cell-like cell to be isolated. In this regard, intestinal epithelial cell-like cells adhere strongly to each other through intercellular adhesion. Thus, in a case where an intestinal epithelial cell-like cell and another cell are in the form of a layered structure, the intestinal epithelial cell-like cell can be detached and isolated from the another cell, using tweezers or the like.

### <Step (D)>

A method of producing a feeder cell according to the present disclosure may further include, in addition to the above-described steps (A) to (C), a step (step (D)) of proliferating the fibroblast-like cell isolated in the step (C).

The fibroblast-like cell isolated in the step (C) can be cultured using a method that is usually used to culture a fibroblast.

A medium to be used in the present step (D) is not particularly limited, subject to being a medium for proliferating a fibroblast. Examples of the medium include an MEM medium, BME medium, DMEM medium, DMEM-F12 medium, aMEM medium, IMDM medium, ES medium, DM-160 medium, Fisher medium, F12 medium, WE medium, and RPMI1640 medium. In addition, to these media, serum or the property of substituting for serum may be added, if desired.

A fibroblast-like cell is cultured usually at 37°C. The culture is preferably performed in an atmosphere having a CO₂ concentration of approximately 5%, using a CO₂ cell culture device or the like.

The fibroblast-like cell is preferably cultured until the fibroblast-like cell is confluent or substantially confluent. The culture period can be suitably set in accordance with the speed of proliferation of the fibroblast-like cell, the shape and size of a culture container to be used, and the like. The culture period of the fibroblast-like cell may be, for example, 3 to 7 days, or 7 to 12 days.

### [Feeder cell]

One aspect of the present disclosure provides a fibroblast-like cell (i.e., a feeder cell according to the present disclosure) for maintaining a cell deemed to be difficult to maintain and proliferate by a two-dimensional culture, examples of which cell include an intestinal epithelial cell. In one embodiment, a feeder cell according to the present disclosure exhibits a specific gene expression profile. In one embodiment, a feeder cell according to the present disclosure is characterized by expressing each of the genes: a platelet-derived growth factor receptor (PDGFRA), CD81, forkhead box L1 (Foxl1), and gremlin-1 (GREM1). In addition, in another embodiment, a feeder cell according to the present disclosure is characterized by expressing each of the genes PDGFRA, CD81, and GREM1, and by not expressing the CD34 gene. In one preferable embodiment, a feeder cell according to the present disclosure is characterized by expressing each of the genes PDGFRA, CD81, Foxl1, and GREM1, and by not expressing the CD34 gene. These feeder cells according to the present disclosure can maintain or proliferate, by a two-dimensional culture, a cell deemed to be difficult to maintain and proliferate by a two-dimensional culture, examples of which cell include an intestinal epithelial cell.

Studies are being advanced, using a mouse, on a feeder cell for maintaining a cell hitherto deemed to be difficult to maintain and proliferate by a two-dimensional culture, examples of which cell include an intestinal epithelial cell. Under the current situation, however, a feeder cell capable of singly maintaining a cell such as an intestinal epithelial cell (i.e., having a complete feeder capability) has not been discovered hitherto, and a plurality of species of feeder cells are used in combination to achieve the maintenance of a cell such as an intestinal epithelial cell. In addition, examples of a typical feeder cell that has been discovered conventionally include a trophocyte of and a telocyte of a mouse. It is known that a trophocyte expresses each of the genes PDGFRA, CD81, and CD34, but on the other hand, does not express the Foxl1 gene. In addition, it is known that a telocyte expresses each of the genes PDGFRA and Foxl1, but on the other hand, expresses none of the genes CD81 and CD34. Accordingly, as with a feeder cell according to the present disclosure, a cell that expresses each of the genes PDGFRA, CD81, Foxl1, and GREM1 and a cell that expresses each of the genes PDGFRA, CD81, and GREM1, but does not express the CD34 gene have not been discovered, in the first place. That such a cell has an excellent feeder capability for a cell such as an intestinal epithelial cell can be said to be a conventionally unknown surprising discovery.

In one embodiment, a feeder cell according to the present disclosure can be obtained by performing the above-described steps (A) to (C) and optionally the step (D). A feeder cell according to the present disclosure can maintain or proliferate, by a two-dimensional culture, a cell hitherto deemed to be difficult to maintain and proliferate by a two-dimensional culture. In particular, a feeder cell according to the present disclosure achieves a feeder capability for a cell as an object over a long period of culture (for example, 10 passages or more), compared with a conventional feeder cell.

A cell as an object (hereinafter also referred to as an "object cell") for which a feeder cell according to the present disclosure is to be used is not particularly limited, and is preferably such a cell difficult to maintain and proliferate by a two-dimensional culture as described above. More specifically, the object cell is preferably a cell prone to cause rapid dedifferentiation owing to a stress due to undergoing a passage or a culture period during a two-dimensional culture. Examples of such an object cell include a small-intestine epithelial cell, large-intestine epithelial cell, hepatocyte, and chondrocyte. In one preferable embodiment, the object cell is a small-intestine epithelial cell or a hepatocyte. Accordingly, in one embodiment, a feeder cell according to the present disclosure is a feeder cell for maintaining or proliferating each of the above-described cells by a two-dimensional culture (plane culture). That is, even in a case where a feeder cell according to the present disclosure is used to subculture an object cell by a two-dimensional culture a plurality of times (for example, three times or more), the feeder cell can inhibit the properties of the object cell from being lost, and proliferate the object cell as long as the object cell maintains the original properties thereof. In this regard, in the present disclosure, a feeder cell and an object cell may be derived from the same individual (the feeder cell is derived from a pluripotent stem cell derived from a specific individual, and the object cell is derived from a pluripotent stem cell or a naturally-occurring cell that are derived from the same individual), or may be derived from different individuals (the feeder cell is derived from a pluripotent stem cell derived from a specific individual, and the object cell is derived from a pluripotent stem cell or a naturally-occurring cell that are derived from another individual of the same species). In this regard, the "naturally-occurring cell" means a cell present in an individual, and in a state that is not artificially manipulated (i.e., in a "naturally-occurring" state). Specific examples include a small-intestine epithelial cell, large-intestine epithelial cell, hepatocyte, and chondrocyte collected from a human.

In one embodiment, the object cell is a cell derived from a pluripotent stem cell, preferably a small-intestine epithelial cell, large-intestine epithelial cell, hepatocyte, or chondrocyte derived from a pluripotent stem cell, more preferably a small-intestine epithelial cell or hepatocyte derived from a pluripotent stem cell.

In a preferable embodiment, the object cell is an intestinal epithelial cell-like cell collected from the above-described intestinal structure, particularly preferably an intestinal epithelial cell-like cell collected from the same intestinal structure as prepared in the step (A) for the purpose of obtaining a feeder cell according to the present disclosure. In such a manner, that both a feeder cell according to the present disclosure and an intestinal epithelial cell-like cell are derived from the same intestinal structure produced from the same pluripotent stem cell provides the below-described advantages. A first advantage is that, because a feeder cell and an intestinal epithelial cell-like cell are derived from the same pluripotent stem cell, the safety of each cell can be verified easily. Specifically, before inducing a pluripotent stem cell to differentiate into each of a feeder cell and an intestinal epithelial cell-like cell, just verifying the safety of the pluripotent stem cell as the origin of each cell makes it possible to verify the safety of each of the feeder cell and the intestinal epithelial cell-like cell that are obtained through the induction of differentiation. In addition, the origin of the feeder cell and the origin of the intestinal epithelial cell-like cell are the same pluripotent stem cell, and thus, a component derived from a different species does not come in after the induction of differentiation. Accordingly, use of one or both of these cells after induction of differentiation does not cause a problem of contamination by a component derived from a different species, and the cells can each be directly used as what is called a "xeno-free" cell in medical applications in particular.

In one embodiment, an intestinal epithelial cell-like cell collected from the above-described intestinal structure expresses at least one intestinal epithelial cell marker selected from the group consisting of CDX2, ECAD, ZO-1, and cytokeratin AE1/AE3. In a preferable embodiment, an intestinal epithelial cell-like cell collected from the above-described intestinal structure expresses at least one intestinal epithelial cell marker selected from the group consisting of CDX2 and Villin.

In a case where an object cell is cultured using a feeder cell according to the present disclosure, the passage number of the feeder cell to be used is not particularly limited, and may be, for example, 2 or more, 3 or more, 5 or more, 7 or more, 10 or more, 12 or more, or 15 or more. On the other hand, the passage number of the feeder cell to be used may be, for example, 20 or less, 15 or less, or 12 or less.

Singly culturing a feeder cell according to the present disclosure makes it possible also to proliferate the feeder cell. Furthermore, coculturing a feeder cell according to the present disclosure and a specific object cell makes it possible to proliferate the feeder cell and the object cell together.

Singly culturing a feeder cell according to the present disclosure makes it possible to obtain a culture product (cell population) containing the feeder cell according to the present disclosure or a culture product composed of the feeder cell according to the present disclosure. Specifically, singly culturing a feeder cell according to the present disclosure two-dimensionally makes it possible to obtain a two-dimensionally-shaped (for example, sheet-like) culture product containing the feeder cell according to the present disclosure or a two-dimensionally-shaped (for example, sheet-like) culture product composed of the feeder cell according to the present disclosure. In this regard, "containing" a specific cell with reference to a "culture product" and a "coculture product" means that a cell other than the specific cell can be contained in the culture product, and to be "composed of" a specific cell means that a cell other than the specific cell is not intentionally contained in the culture product.

In addition, as described above, a feeder cell according to the present disclosure can maintain or proliferate, by a two-dimensional culture, an object cell, particularly a cell deemed to be difficult to maintain and proliferate by a two-dimensional culture. Accordingly, using a feeder cell according to the present invention to culture an object cell (i.e., coculturing the feeder cell according to the present disclosure and the object cell) makes it possible to obtain a two-dimensionally-shaped (for example, sheet-like) coculture product containing the feeder cell according to the present invention and the object cell or a two-dimensionally-shaped (for example, sheet-like) coculture product composed of the feeder cell according to the present disclosure and the object cell. In particular, using a feeder cell according to the present disclosure makes it possible to two-dimensionally maintain or proliferate a cell hitherto deemed to be difficult to maintain and proliferate by a two-dimensional culture, and thus, makes it possible to obtain even such a cell in the form of a two-dimensionally-shaped (for example, sheet-like) coculture product. For example, such a cell can be obtained in the form of a coculture product containing a layer of the feeder cell and a layer of the object cell. In addition, removing part or the whole of a feeder cell according to the present disclosure by separation from such a coculture product makes it possible to obtain a culture product containing an object cell or a culture product composed of the object cell. Specifically, a feeder cell according to the present disclosure is made sheet-like. On the sheet, an object cell deemed to be difficult to maintain and proliferate by a two-dimensional culture is cocultured and maintained or proliferated, thereby a two-dimensionally-shaped (for example, sheet-like) coculture product of the object cell is obtained, and then, from the resulting coculture product, part or the whole of the feeder cell according to the present disclosure is removed by separation, a two-dimensionally-shaped culture product containing the object cell or a two-dimensionally-shaped culture product composed of the object cell can be obtained. On the other hand, removing, by separation, part or the whole of the object cell from the coculture product obtained as described above also makes it possible to obtain a two-dimensionally-shaped culture product containing a feeder cell according to the present disclosure or a two-dimensionally-shaped culture product composed of the feeder cell according to the present disclosure.

In one embodiment, the object cell is a cell deemed to be difficult to maintain and proliferate by a two-dimensional culture, examples of which cell include an intestinal epithelial cell (for example, small-intestine epithelial cell or large-intestine epithelial cell), hepatocyte, or chondrocyte. The object cell is preferably an intestinal epithelial cell.

A feeder cell according to the present disclosure and an object cell that are contained in a coculture product can usually be separated using a known method used in the field of cell culture. In one embodiment, in a case where a coculture product contains a feeder cell according to the present disclosure and an intestinal epithelial cell-like cell, the intestinal epithelial cell-like cell is isolated from the coculture product using a method disclosed in the pamphlet of WO2019/131938.

In one embodiment, such a coculture product of a feeder cell according to the present disclosure and an object cell, a coculture product containing the object cell, and a coculture product containing the feeder cell can each be used as what is called a cell sheet. A cell sheet is a thin membrane produced by culturing a cell in sheet form, and has advantages such as the capability of being transplanted into an organism tissue without use of a surgical suture and the capability of being transplanted in a short time. Utilizing such characteristics, the cell sheet is used for treatment of various diseases and for tissue regeneration. Accordingly, in one embodiment, a cell sheet is used for treatment of a disease and as an implant for tissue regeneration.

As described above, a feeder cell according to the present disclosure makes it possible to maintain or proliferate an object cell by a two-dimensional culture, and thus, makes it possible to easily produce a two-dimensionally-shaped coculture product such as a cell sheet. In addition, suitably setting the size and shape of a culture container for coculturing a feeder cell according to the present disclosure and an object cell, the culture time, and the like makes it possible to produce a cell sheet having a desired size and a two-dimensional shape. Conventionally, a cell such as an intestinal epithelial cell has been difficult to maintain and proliferate by a two-dimensional culture, and thus, it has also not been easy to produce a two-dimensionally-shaped coculture product such as a cell sheet mainly containing such a cell. Accordingly, to produce, for example, a large cell sheet or the like, there has been no choice but to use a combination of a plurality of relatively small two-dimensionally-shaped cell sheets that are producible. On the other hand, using a feeder cell according to the present disclosure makes it possible that such a cell is maintained or proliferated by a two-dimensional culture, that the size and shape of the cell sheet are suitably set, and that a large cell sheet is produced without combining a plurality of cell sheets as described above. For example, using a feeder cell according to the present disclosure makes it possible that a very large cell sheet is produced in the form of one cell sheet without combining a plurality of cell sheets, in which the very large cell sheet is a circle or ellipse having a diameter of 1 cm or more, 2 cm or more, 3 cm or more, 5 cm or more, or 10 cm or more, or a polygon (for example, quadrangle) having approximately the same size. In such a manner, a cell hitherto deemed to be difficult to maintain and proliferate by a two-dimensional culture can be used to produce a large cell sheet without combining a plurality of cell sheets. This fact is one of the excellent effects brought about by a feeder cell according to the present disclosure. For example, in the case of Crohn's disease, the pathological condition of the disease is judged using the size (diameter) of an ulcer as an index. In a case where the diameter is 2 cm or more, the disease is judged difficult to heal. Accordingly, it can be said that efficiently producing such a very large cell sheet in the form of one cell sheet can greatly contribute to treating, for example, Crohn's disease that is effectively treated using a cell sheet.

In addition, there is a conventionally known method in which a precursor cell in a stage before differentiation into a specific cell is produced from a pluripotent stem cell or the like, and proliferated by a two-dimensional culture using a proliferation medium, and then, differentiation of the precursor cell is promoted by a differentiation medium, whereby a structure constituted by a functional cell is obtained. Here, in a case where a cell of interest is a cell prone to cause rapid dedifferentiation owing to a stress due to undergoing a passage or a culture period during a two-dimensional culture, the dedifferentiation occurs in a stage of differentiation promotion of a differentiation medium, and thus, the property of the cell of interest usually cannot be maintained for a long period of time. That is, a structure in which the property of the cell of interest is maintained for a long period of time can not be obtained. However, using a feeder cell according to the present disclosure can inhibit such dedifferentiation in the stage of promotion of differentiation, and makes it possible to maintain the property of the cell of interest for a long period of time. Accordingly, a feeder cell according to the present disclosure can be suitably used in such an application as a structure (for example, implant or drug development tool) in which the property of the cell of interest needs to be maintained for a long period of time.

In addition, a feeder cell according to the present disclosure can be produced so as to be free from a component derived from a different species (what is called, under a xeno-free condition). Accordingly, in a case where the feeder cell is used for an implant such as the above-described cell sheet, a risk due to a component derived from a different species can be eliminated in transplantation. In addition, as shown in Examples, a primary intestinal epithelial cell collected from a human was able to be proliferated using a feeder cell according to the present disclosure as a foothold. Thus, it is inferred that single transplantation of a feeder cell according to the present disclosure can contribute to prompt autologous tissue regeneration.

In addition, a feeder cell according to the present disclosure conceivably has various functions besides such functions as described above as being characteristic of a feeder cell. Accordingly, the application of a feeder cell according to the present disclosure is not limited to such an application as characteristic of a feeder cell. The feeder cell can also be used in an application in which a function different from a function characteristic of a feeder cell is expected.

For example, one aspect of the present disclosure provides a pharmaceutical composition containing a feeder cell according to the present disclosure (fibroblast-like cell) or a coculture product of a feeder cell (fibroblast-like cell) and, for example, various cells, such as a small-intestine epithelial cell and a hepatocyte. Here, a pharmaceutical composition is obtained by preparing a feeder cell (fibroblast-like cell) or a coculture product according to the present disclosure in the form of an oral preparation or a parenteral preparation in accordance with an ordinary method. For preparation, an additive acceptable for preparation may be used in combination. Examples of the additive acceptable for preparation include an excipient, stabilizer, antiseptic, wetting agent, emulsifier, lubricant, sweetener, coloring agent, perfume, buffering agent, antioxidant, and pH adjustor. A pharmaceutical composition that is an oral preparation can be in the form of a solid preparation, such as a tablet, powder, fine granule, granule, capsule, pill, or sustained-release preparation, or a liquid preparation, such as a solution, suspension, or emulsion. In addition, a pharmaceutical composition that is a parenteral preparation can be in the form of an injection, suppository, sheet-like patch, suspension for application, spray, or the like.

The amount of administration (application) of a pharmaceutical composition is not particularly limited, subject to achieving the effects of the present disclosure, and can be suitably adjusted in accordance with the age, health condition, body weight, and the like of a subject of administration (application). For example, in a case where a feeder cell according to the present disclosure is used in the form of a sheet-like patch to treat an ulcer of a mucosa in a subject, the sheet-like patch is applied so as to completely cover the ulcerous portion and the portion therearound. In a case where the ulcer is large, a plurality of patches are combined, and the patches are applied in combination so as to completely cover the ulcerous portion and the portion therearound.

A feeder cell (fibroblast-like cell) or a coculture product is preferably ingested (administered) continuously over a long period of time to achieve the effects thereof better. The period of ingestion (administration) can be, for example, 1 to 6 weeks, 1 to 12 weeks, 2 to 10 weeks, 4 to 10 weeks, or 4 to 12 weeks. As used herein, the term "continuously" means continuing to ingest (administer) a pharmaceutical composition in a predetermined amount every day.

Another aspect of the present disclosure provides a method of treating a disease of a subject, the method including allowing an effective amount of a feeder cell (fibroblast-like cell) or a coculture product to be ingested by (administered to) the subject. The method may be a nontherapeutic method, or may be a therapeutic method.

In the method of treating a disease, the amount of administration and period of ingestion (administration) of a feeder cell (fibroblast-like cell) or a coculture product is not particularly limited, subject to achieving the effects of the present disclosure, and can be suitably adjusted in accordance with the kind of the disease and the age, health condition, body weight, and the like of a subject of ingestion (administration).

Another aspect of the present disclosure provides a method of treating a disease of a subject, the method including applying the above-described cell sheet to the subject. The method may be a nontherapeutic method, or may be a therapeutic method.

In the method of treating a disease, the shape and size of a cell sheet is not particularly limited, subject to achieving the effects of the present disclosure, and can be suitably adjusted, for example, in accordance with the kind of the disease, the kind of an organ or tissue as a subject of application, and the shape and size of a portion as a subject of application.

Another aspect of the present disclosure provides use of a feeder cell (fibroblast-like cell) or a coculture product according to the present disclosure for the purpose of producing a composition for treating a disease.

In each of the above-described aspects, examples of a disease that can be treated using a feeder cell (fibroblast-like cell), coculture product, or cell sheet according to the present disclosure include: Crohn's disease; a disease caused by an ulcer of a digestive tract, such as ulcerative colitis; and a complication caused by an operation on a digestive tract, such as caused after an operation for colon cancer.

In each of the above-described aspects, a cell sheet containing a feeder cell (fibroblast-like cell) according to the present disclosure, a cell sheet containing the feeder cell (fibroblast-like cell) and a coculture product, and a cell sheet containing the coculture product can be used in an application of transplantation into a body of a human or a nonhuman animal, particularly in an application of coverage of an inflamed site, wound site, lesion site, postoperative sutured site, abdominal cavity, or missing intestinal tissue in an organ or the like.

In particular, in a case where a cell sheet (i.e., a cell sheet according to the present disclosure) containing a feeder cell (fibroblast-like cell) according to the present disclosure has a mucus-secreting capability, specifically a mucin-secreting capability, the cell sheet according to the present disclosure is highly effective in protecting a portion (for example, an agglutinated site or the like in an ulcer or the like) having the cell sheet applied thereto, and thus, is preferable. A cell sheet according to the present disclosure is preferably applied to, and agglutinates, an inflamed site, wound site, lesion site, postoperative sutured site, abdominal cavity, or the like, whereby the site of application can be protected.

A cell sheet according to the present disclosure is preferably agglutinated to an inflamed site, wound site, lesion site, postoperative sutured site, or abdominal cavity, and thereby can be inhibited from leaking into the agglutinated site, leaking from the agglutinated site, or invading into the agglutinated site.

A cell sheet according to the present disclosure is preferably agglutinated to an inflamed site, wound site, lesion site, postoperative sutured site, or abdominal cavity, whereby a cell necessary to heal the agglutinated site and a foothold for the cell can be provided.

A cell sheet according to the present disclosure is preferably agglutinated to an inflamed site, wound site, lesion site, postoperative sutured site, or abdominal cavity, and thereby can prevent the agglutinated site from adhering to an ectopia.

A cell sheet according to the present disclosure is preferably agglutinated to an inflamed site, wound site, lesion site, postoperative sutured site, or abdominal cavity, and thereby can inhibit an immunoreaction, for example, can inhibit infiltration of a hematopoietic cell into a tissue of a digestive tract, in which the infiltration is caused by an autologous immunoreaction.

A cell sheet according to the present disclosure is preferably agglutinated to an inflamed site, wound site, lesion site, postoperative sutured site, or abdominal cavity, and thereby can mitigate enhancement of inflammation of an agglutinated site. A cell sheet according to the present disclosure is preferably agglutinated to a postoperative sutured site, and thereby can prevent a postoperative complication. A cell sheet according to the present disclosure is preferably transplanted into a missing intestinal tissue, and thereby can substitute to perform an intestine function.

A cell sheet according to the present disclosure can be held with tweezers or the like, and attached to a desired site of implantation in any of an endoscopic operation, laparotomy, laparoscopic operation, and the like. In a case where a cell sheet according to the present disclosure is transplanted into a site into which transplantation is difficult in a laparoscopic operation or the like, the cell sheet may be transplanted integrally with a support, or may be held with a carrier such as a wire, and transplanted. A cell sheet according to the present disclosure can be fixed on a biocompatible support, and transplanted together with a support.

In a case where a cell sheet according to the present disclosure is transplanted into an organism, a biocompatible adhesive auxiliary substance, such as fibrin, may be applied to a cell sheet according to the present disclosure and/or a site of implantation. In addition, a cell sheet according to the present disclosure and a site of implantation may be sutured.

In a case where a cell sheet according to the present disclosure is attached to a site of implantation, the cell sheet according to the present disclosure may be, for example, widened with tweezers, mounted on a site of implantation, and attached, or the cell sheet according to the present disclosure may be mounted on a site of implantation, and then widened. Alternatively, using a jig having a balloon structure, a cell sheet according to the present disclosure can also be pressed to a site of implantation to be attached thereto.

Another aspect of the present disclosure relates to the following [1] to [28].
[1] A method of producing a feeder cell for maintaining or proliferating a cell, including:
   (A) a step of preparing an intestinal structure produced from a pluripotent stem cell, and containing an endoderm-derived cell and a mesoderm-derived cell;
   (B) a step of culturing the intestinal structure to proliferate a fibroblast-like cell derived from the intestinal structure; and
   (C) a step of isolating the fibroblast-like cell to obtain a feeder cell.
[2] The method according to [1], wherein the intestinal structure is produced using a method including:
   (a) a step of preparing a cell culture substrate including: a substrate; and a plurality of isolated cell adhesion regions formed on the substrate, and cell non-adhesion regions that surround each of the cell adhesion regions;
   (b) a step of seeding a pluripotent stem cell on the cell culture substrate; and
   (c) a step of culturing, in a medium, the pluripotent stem cell seeded.
[3] The method according to [1] or [2], further including (D) a step of proliferating the fibroblast-like cell isolated.
[4] The method according to any one of [1] to [3], wherein, in the step (B), the intestinal structure is cultured in a culture container, and at least part of the intestinal structure is in contact with the culture container.
[5] The method according to [2], wherein a culture period in the step (c) is 60 days or more.
[6] A feeder cell produced using the method according to any one of [1] to [5].
[7] A fibroblast-like cell which is positive for PDGFRA and CD81, and expresses Foxl1 and GREM1.
[8] The fibroblast-like cell according to [7], which is positive for PDGFRA and CD81, is negative for CD34, and expresses Foxl1 and GREM1.
[9] The fibroblast-like cell according to [7] or [8], which is derived from a culture product of an intestinal structure that is derived from a pluripotent stem cell or produced from a pluripotent stem cell, and contains an endoderm-derived cell and a mesoderm-derived cell.
[10] The method according to [9], wherein the intestinal structure is produced using a method including:
   (a) a step of preparing a cell culture substrate including: a substrate; and a plurality of isolated cell adhesion regions formed on the substrate, and cell non-adhesion regions that surround each of the cell adhesion regions;
   (b) a step of seeding a pluripotent stem cell on the cell culture substrate; and
   (c) a step of culturing, in a medium, the pluripotent stem cell seeded.
[11] The fibroblast-like cell according to [9] or [10], wherein the pluripotent stem cell is human-derived.
[12] The feeder cell according to [6] or the fibroblast-like cell according to any one of [7] to [11], which is a feeder cell for maintaining or proliferating at least one cell selected from the group consisting of a small-intestine epithelial cell, large-intestine epithelial cell, hepatocyte, and chondrocyte.
[13] The feeder cell according to [6] or the fibroblast-like cell according to any one of [7] to [12], which is a feeder cell for maintaining or proliferating the at least one cell by a two-dimensional culture.
[14] The feeder cell according to [6] or the fibroblast-like cell according to any one of [7] to [13], which is a feeder cell capable of subculturing the at least one cell three times or more.
[15] A cell sheet including the feeder cell according to [6] or the fibroblast-like cell according to any one of [7] to [14].
[16] A coculture product including: at least one cell selected from the group consisting of a small-intestine epithelial cell and a hepatocyte; and the feeder cell according to [6] or the fibroblast-like cell according to any one of [7]to [14].
[17] The coculture product according to [16], the passage number of the at least one cell is 3 or more.
[18] A cell sheet including the coculture product according to [16] or [17].
[19] The cell sheet according to [18], having a circular or generally circular shape having a diameter of 1 cm or more.
[20] The cell sheet according to [18] or [19], including a layer of at least one cell selected from the group consisting of a small-intestine epithelial cell and a hepatocyte.
[21] A cell sheet according to any one of [18] to [20], further containing a layer of the feeder cell.
[22] An implant containing the cell sheet according to any one of [15] and [18] to [21].
[23] A method of maintaining or proliferating a cell, including a step of culturing the cell in the presence of the feeder cell according to [6] or the fibroblast-like cell according to any one of [7] to [14].
[24] The method of maintaining or proliferating a cell according to [23], wherein a culture for the cell is a two-dimensional culture.
[25] The method of maintaining or proliferating a cell according to [23] or [24], wherein the cell is at least one cell selected from the group consisting of a small-intestine epithelial cell, large-intestine epithelial cell, hepatocyte, and chondrocyte.
[26] The method of maintaining or proliferating a cell according to any one of [23] to [25], wherein the cell and the feeder cell are derived from the same individual.
[27] The method of maintaining or proliferating a cell according to any one of [23] to [26], wherein the cell and the feeder cell are derived from different individuals.
[28] A cell which expresses at least one gene selected from the group consisting of DCN, ACTA2, and NOG, and expresses the genes, ADAMTS19, ANO1, BLID, LOC100507053, LHX8, SFRP4, AHRR, SLC14A1, FMO3, PZP, ABCG4, EYA2, TMEM92-AS1, ANO10, EGFL6, SNCAIP, LGSN, PCDHB15, and KRTAP1-5.

### Examples

The present disclosure will be described below in further detail with reference to Examples, and the present disclosure is not limited to these Examples.

### [Example 1] Production of cell culture substrate

Toluene in an amount of 39.0 g and 13.5 g of methacryloyl silane TSL8370 (manufactured by GE Toshiba Silicone Co., Ltd.) were mixed. To the resulting mixture, 450 µl of triethylamine was added with stirring. The resulting solution was directly stirred at room temperature for several minutes, and then, the whole amount of the solution was transferred to a glass dish. In the resulting solution, a 5 cm square glass base plate cleaned with UV was immersed and left to stand at room temperature for 16 hours. Then, the glass base plate was cleaned with ethanol and water, and dried with a nitrogen blow. In this manner, a thin film containing a methacryloyl group was formed on the surface of the glass base plate.

In 10 g of polyethylene glycol diacrylate (PEGdA, manufactured by Sigma-Aldrich), 0.1 g of 2,2'-dimethoxy-2-phenyl-acetophenone (DMPA, manufactured by Sigma-Aldrich) as a polymerization initiator was dissolved at room temperature. With this resulting solution, the above-described methacryloylated base plate was spincoated at 1500 rpm for 5 seconds. Then, the whole surface of the base plate was irradiated rapidly with ultraviolet rays under a nitrogen atmosphere for 3 seconds. Then, the resulting base plate was post-baked at 160°C for 10 minutes. This resulting PEGdA-coated base plate was immersed in water overnight, then washed with water, and then dried. Here, the average thickness of the dried film was 0.33 µm.

A photomask 5 inches in size was used, the photomask having a pattern in which a plurality of circular openings each having a diameter of 1.5 mm were formed. All the spaces each between one opening and another in the photomask, that is, the shortest distances each between one opening and another, were each 0.35 mm.

The mask was calmly mounted on the PEGdA side of the PEGdA-coated base plate, and the reverse side of the mask was irradiated for 1 minute with vacuum ultraviolet rays from a xenon eximer lamp (172 nm, 10 mW/cm²) used as a light source. In this manner, the regions corresponding to the openings of the photomask on the surface of the PEGdA film were oxidized. Then, the base plate was cut to 2.5 cm square, and used as a cell culture substrate.

The shape of a cell adhesion region in the resulting cell culture substrate was circular, and the diameter of the region was 1.5 mm. All the spaces each between one cell adhesion region and another, that is, the shortest distances each between one cell adhesion region and another, were each 0.35 mm.

### [Example 2] Production of intestinal structure

On the surface of the cell culture substrate produced in Example 1, pluripotent stem cells (SEES-2 cells that are in an ES cell line established by National Center for Child Health and Development, a National Research and Development Agency, Japan) were seeded at approximately 1 × 10⁷ cells/cm², cultured under conditions at 37°C and at a CO₂ concentration of 5% in an incubator for 60 days, and thereby induced to differentiate. The pluripotent stem cells were cultured using an XF32 medium. After the induction of differentiation for 60 days, a cell suspension-gelling reagent (iPGell, manufactured by Genostaff Co., Ltd.) was used to embed the culture product in gel. The culture product embedded in gel was cut to produce a culture product section. The composition of the XF32 medium used to culture the pluripotent stem cells is shown in Table 1 below.

**[Table 1]**

| Component (tradename) | Manufacturer | Amount added per L | Final concentration |
|---|---|---|---|
| D-MEM free from animal-derived component (KnockOut (trademark) D-MEM) | Life Technologies | 810 mL | 81% (v/v) |
| Serum replacement free from animal-derived component (KnockOut (trademark) serum replacement XenoFree) | | 150 mL | 15% (v/v) |
| MEM Non-Essential Amino Acids Solution 100 mM, liquid | | 10 mL | 0.1 mM |
| Sodium Pyruvate Solution 100 mM, liquid | | 10 mL | 1 mM |
| L-glutamine replacement GlutaMAX (trademark) Supplement 200 mM, liquid | | 10 mL | 2 mM |
| Penicillin-Streptomycin, liquid (100x) | | 10 mL | |
| Ascorbic acid | Sigma-Aldrich | 50 ng | 50 µg/mL |
| Human basic fibroblast growth factor (HU FGF BASIC FULL LENGTH 100 µg) | Life Technologies | 20 µg | 20 ng/mL |
| Heregulin-β1 | Fujifilm Wako Pure Chemical Corporation | 10 µg | 10 ng/mL |
| Insulin-like growth factor 1 | Nichirei Corporation | 200 µg | 200 ng/mL |

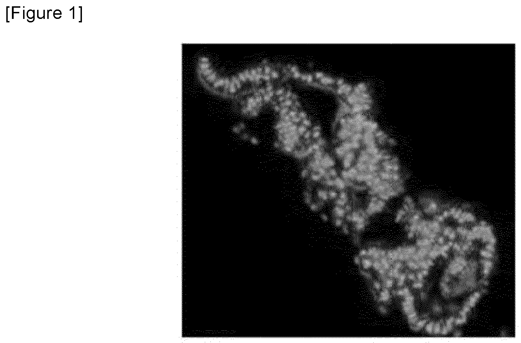

The culture product section obtained was immunostained using an antibody against each of Villin and 5TH, differentiation markers for an intestine. As a result, Villin and 5TH were detected in the cell adhesion regions to verify that a saccate structure (i.e., intestinal structure) had been formed (FIG. 1). Furthermore, it has been verified that the saccate structure made a peristaltic movement, and thus, it has been verified that a cell necessary for a peristaltic movement, and derived from an endoderm, mesoderm, and ectoderm was present. In addition, as the result of observation under a stereoscopic microscope, a peristaltic-movement-like movement in the saccate structure was observed, and the presence of a Cajal cell that is an ectoderm, an intestinal epithelial cell that is an endoderm, and a smooth muscle that is a mesoderm was verified.

### [Example 3] Maintenance and proliferation 1 of fibroblast-like cell and intestinal epithelial cell that are derived from intestinal structure

An intestinal structure produced in Example 2 was transferred to another adhesive culture dish, and cultured under conditions at 37° C and at a CO₂ concentration of 5% in an incubator for 17 days to proliferate a fibroblast-like cell and an intestinal epithelial cell-like cell that were derived from the intestinal structure. To culture the intestinal structure, an XF32 medium was used. In this regard, to culture the intestinal structure, the amount of the medium was regulated in such a manner that at least part of the intestinal structure was always in contact with the bottom face of the culture container.

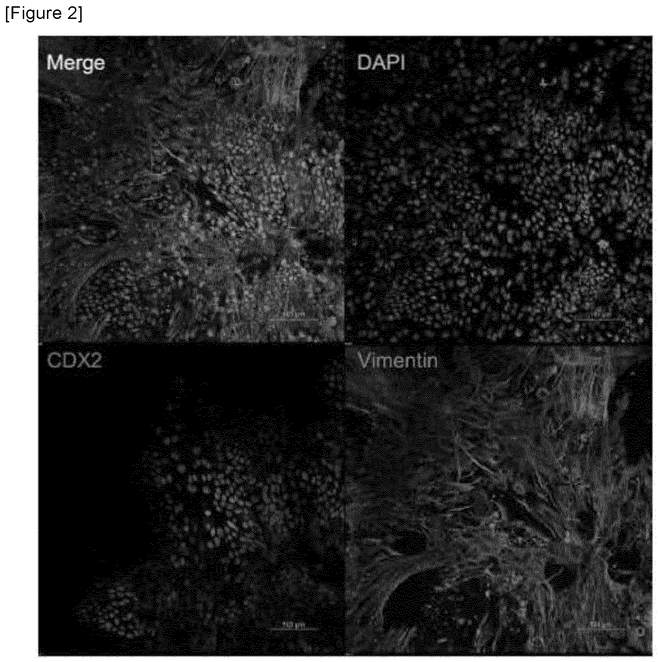

Only the fibroblast-like cell obtained was cultured to expand by a twice-per-week medium exchange and an every-week passage, using D-MEM supplemented with FBS at 10%. A large stock having a passage number of 7 was thus obtained. In this regard, the stock obtained was separated into two stocks, and each stock was stored in a freezer at -80° C or in a gas phase in a tank containing liquid nitrogen. The stock stored in the tank containing liquid nitrogen was transferred to a freezer at -80° C, and then used. These fibroblast-like cell and intestinal epithelial cell-like cell were cultured together for 5 days. The culture was performed using a procedure in which the fibroblast-like cell was preliminarily cultured for approximately 1 week in D-MEM supplemented with FBS at 10%, and then, the intestinal epithelial cell-like cell was seeded on the **fibroblast-like** cell. For the culture performed after the intestinal epithelial cell-like cell was seeded, an ESTEM-HE medium (GlycoTechnica, Ltd.) containing R-spondin 1 and Wnt3a was used. A coculture product of the intestinal epithelial cell-like cell cultured for 21 days (3 passages) and the fibroblast-like cell cultured for 1 week with exchange at every passage, and having a passage number of 7 was immunostained using an antibody against each of CDX2 as a marker for an intestinal epithelial cell and Vimentin as a marker for a fibroblast. As a result, CDX2 and Vimentin were detected (FIG. 2). In this regard, in FIG. 2, the lower left photograph shows a photograph of immunostaining of CDX2, the upper right photograph shows staining by DAPI, the lower right shows a photograph of immunostaining performed using an antibody against Vimentin, and the upper left shows a photograph in which the three photographs are merged. In addition, in each photograph in FIG. 2, the scale bar denotes 100 *µ* m. The photographs in FIG. 2 have revealed that coculturing a fibroblast-like cell and an intestinal epithelial cell-like cell allowed the intestinal epithelial cell-like cell to be maintained and proliferated. In this regard, Vimentin is a marker for a mesoderm, and an intestinal epithelial cell having CDX2 and Villin expressed therein is recognized as being derived from an endoderm. Thus, it can be said that FIG. 1 and FIG. 2 support the presence of a cell derived from each of an endoderm and a mesoderm in the intestinal structure.

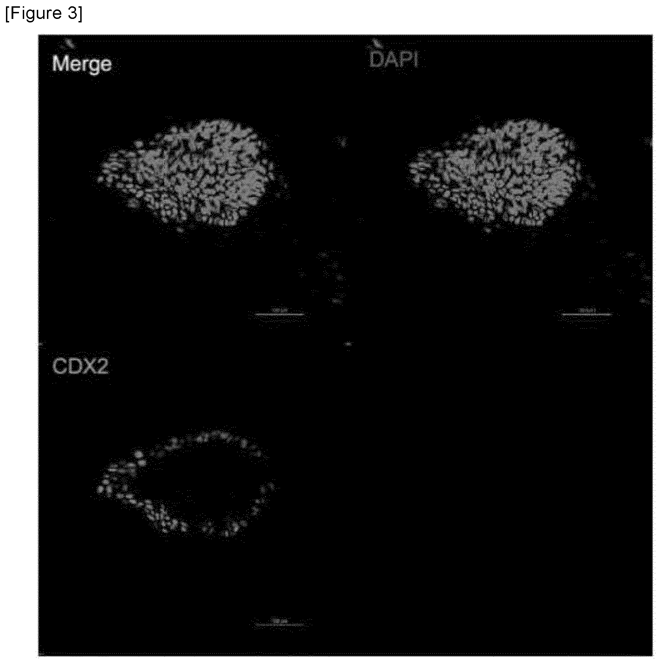

On the other hand, the intestinal epithelial cell-like cell obtained was singly cultured for 2 passages for 14 days. For the culture, the above-described ESTEM-HE medium was used. The culture product after being cultured (for 2 passages) for 14 days was immunostained using an antibody against CDX2, a marker for an intestinal epithelial cell. As a result, CDX2 was detected only in extremely limited cells (FIG. 3). In this regard, in FIG. 3, the lower left photograph shows a photograph of immunostaining of CDX2, the upper right photograph shows staining by DAPI, and the upper left shows a photograph in which the two photographs are merged. In addition, in each photograph in FIG. 3, the scale bar denotes 100 *µ* m. The photographs in FIG. 3 have revealed that singly culturing an intestinal epithelial cell-like cell did not allow the intestinal epithelial cell-like cell to be maintained and that a cell having the property of an intestinal epithelial cell-like cell was not enabled to be proliferated.

### [Example 4] Maintenance and proliferation 2 of fibroblast-like cell and intestinal epithelial cell-like cell that are derived from intestinal structure

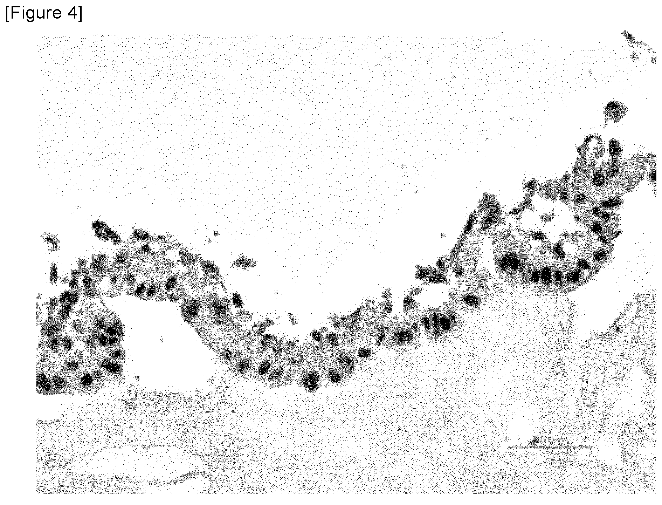

A fibroblast-like cell and an intestinal epithelial cell-like cell that were produced using the same method as in Example 3, and derived from an intestinal structure were cocultured. Specifically, an intestinal epithelial cell-like cell having a passage number of 12 and a fibroblast-like cell having a passage number of 7 were cocultured two-dimensionally for 7 days. For the culture, an ESTEM-HE medium was used. The coculture product was embedded in IP gel. The coculture product embedded and fixed in the IP gel was sliced to obtain a coculture product section. The section obtained was immunostained using an antibody against CDX2, a marker for an intestinal epithelial cell. As a result, CDX2 was detected (FIG. 4). In this regard, in the photograph in FIG. 4, the scale bar denotes 50 *µ* m.

### [Example 5] Cell polarity of fibroblast-like cell and intestinal epithelial cell that are derived from intestinal structure

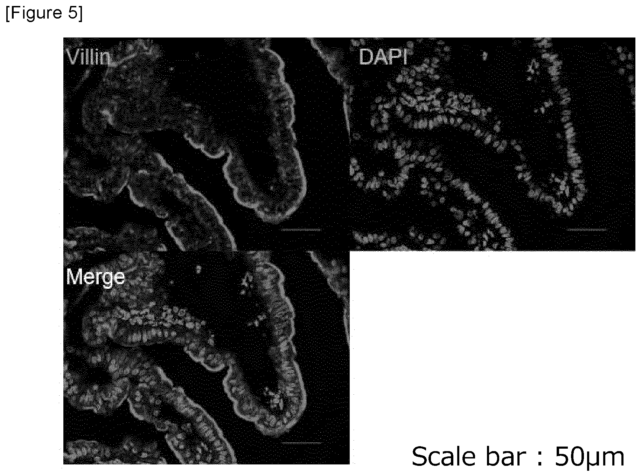

A fibroblast-like cell and an intestinal epithelial cell-like cell that were produced using the same method as in Example 3, and derived from an intestinal structure were cocultured. Specifically, an intestinal epithelial cell-like cell having a passage number of 12 and a fibroblast-like cell having a passage number of 7 were cocultured two-dimensionally for 7 days. For the culture, an ESTEM-HE medium was used. The coculture product was embedded in IP gel. The coculture product embedded and fixed in the IP gel was sliced to obtain a coculture product section. The section obtained was immunostained using an antibody against Villin, a marker for an intestinal epithelial cell. As a result, Villin was detected (FIG. 5). Furthermore, it was verified that Villin was locally present on the lumen side (i.e., Apical side) in an intestinal epithelium of an organism (FIG. 5). This localization is the same as the localization of Villin in an intestinal epithelium of an organism. In this regard, in FIG. 5, the upper left photograph shows a photograph of immunostaining of Villin, the upper right photograph shows staining by DAPI, and the lower left shows a photograph in which the two photographs are merged. In addition, in each photograph in FIG. 5, the scale bar denotes 50 *µ* m.

### [Example 6] Maintenance of intestinal epithelial cell by fibroblast-like cell derived from intestinal structure

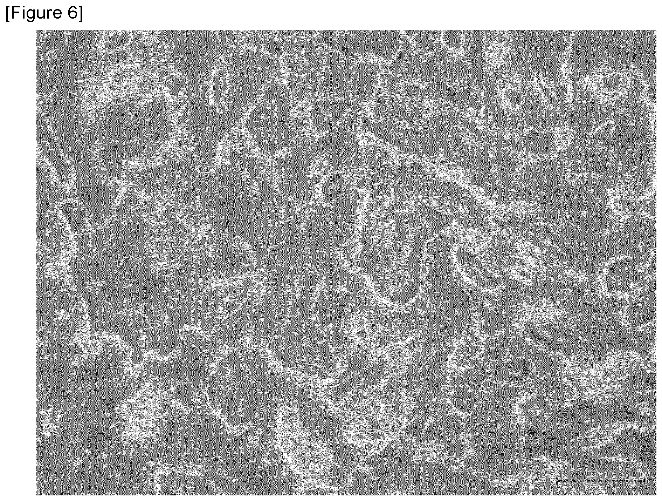

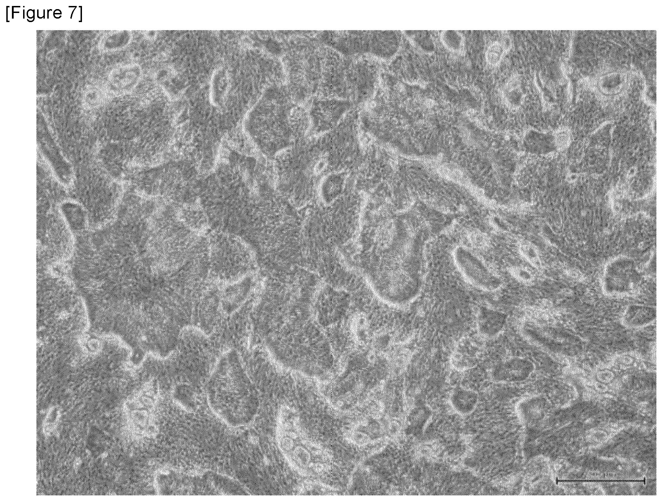

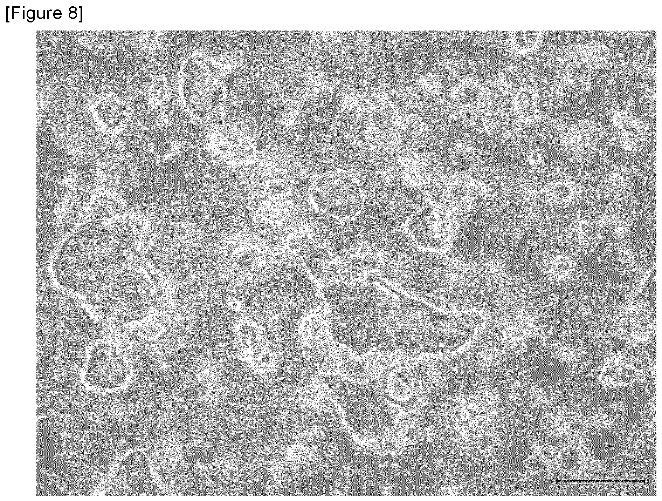

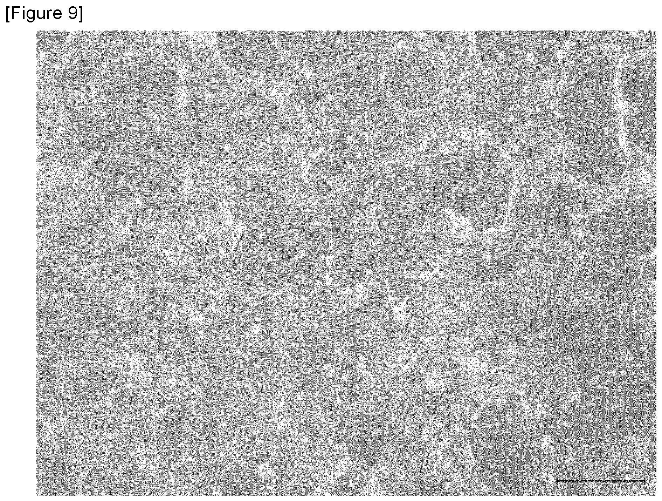

A fibroblast-like cell produced using the same method as in Example 3, derived from an intestinal structure, and having a passage number of 7 and a primary intestinal epithelial cell collected from a human were cocultured. The culture was performed using a procedure in which the fibroblast-like cell was preliminarily cultured for approximately 1 week in D-MEM supplemented with FBS at 10%, and then, the intestinal epithelial cell was seeded on the fibroblast-like cell. For the culture performed after the intestinal epithelial cell was seeded, an ESTEM-HE medium was used. The photomicrograph of the cell at the point of time of each of the passage number 2 (culture day 5) and the passage number 4 (culture day 4) is shown in FIGs. 6 and 7. On the other hand, a primary intestinal epithelial cell collected from a human was cocultured in the same manner as under the above-described conditions except that a mouse embryonic fibroblast (MEF) that is a feeder cell commonly used was used as a feeder cell in place of a fibroblast-like cell derived from an intestinal structure. The photomicrograph of the cell at the point of time of each of the passage number 2 (culture day 5) and the passage number 4 (culture day 4) is shown in FIGs. 8 and 9. In this regard, in each photograph in FIGs. 6 to 9, the scale bar denotes 100 *µ* m.

Comparison between FIGs. 6 and 7 has revealed that the fibroblast-like cell derived from an intestinal structure made it possible to maintain and proliferate the primary intestinal epithelial cell collected from a human. In particular, it is a point worthy of special mention that a primary intestinal epithelial cell that is not an intestinal epithelial cell derived from an intestinal structure can be maintained and proliferated.

On the other hand, comparison between FIGs. 8 and 9 has revealed that some feeder cells that are each not a fibroblast-like cell derived from an intestinal structure did not make it possible to maintain and proliferate the primary intestinal epithelial cell collected from a human. In particular, in FIG. 9, the cells were clearly changed into oblate shape. This is a typical change in the case of dedifferentiation.

### [Example 7] Maintenance of hepatocyte by fibroblast-like cell derived from intestinal structure

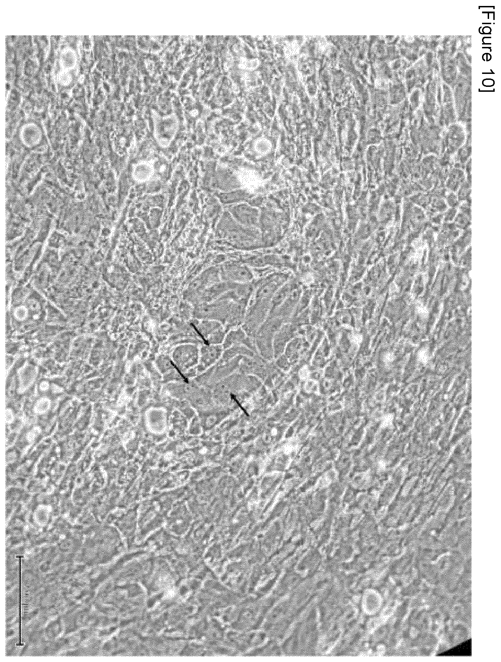

A fibroblast-like cell produced using the same method as in Example 3, derived from an intestinal structure, and having a passage number of 7 and a hepatocyte were cocultured. The culture was performed using a procedure in which the fibroblast-like cell was preliminarily cultured for approximately 1 week in D-MEM supplemented with FBS at 10, and then, the hepatocyte was seeded on the fibroblast-like cell. For the culture performed after the hepatocyte was seeded, an ESTEM-HE medium was used. The photomicrograph of the cell on the 8th day after the start of culture is shown in FIG. 10.

In the center of the photograph in FIG. 10, the diversified cells (denoted by the arrows) peculiar to a stem cell are observed, thus revealing that the fibroblast-like cell derived from an intestinal structure made it possible to maintain and proliferate a hepatocyte.

In addition, a culture of a hepatocyte usually necessitates suitable coating of a culture container and a feeder cell such as a mouse embryonic fibroblast (MEF), but it has been revealed that, without the necessity of these, using a feeder cell according to the present disclosure made it possible to culture a hepatocyte. This result suggests that a feeder cell according to the present disclosure is excellent as a foothold of a culture, and suggests an application in which only an extracellular matrix (ECM) produced by a feeder cell according to the present disclosure can be isolated using a technique such as a decellularization treatment, and used.

### [Example 8] Gene analysis 1 of fibroblast-like cell derived from intestinal structure

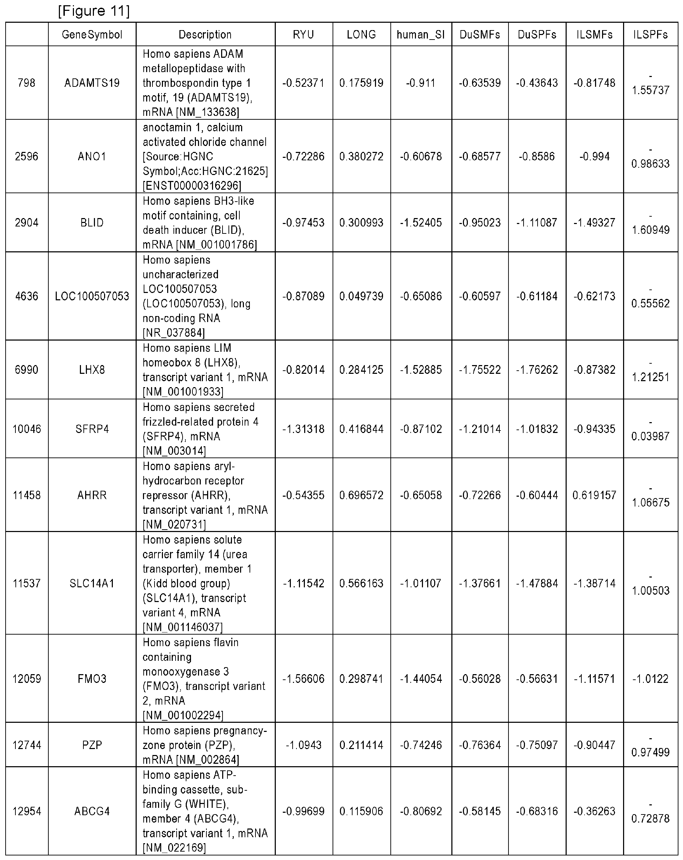

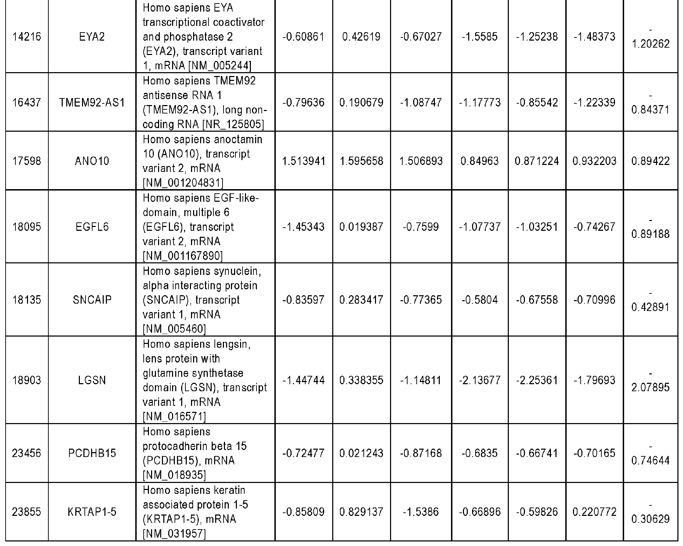

The gene expression of an intestinal tract and a site-specific cell constituting an intestinal tract was analyzed with respect to a fibroblast-like cell and an intestinal epithelial cell-like cell that were derived from an intestinal structure, and produced using the same method as in Example 3. Furthermore, the gene expression with various kinds of fibroblasts in an organism was analyzed. The results are shown in FIG. 11. In FIG. 11, "RYU" and "LONG" represent the gene expression of an intestinal epithelial cell-like cell and a fibroblast-like cell respectively that were derived from an intestinal structure, and produced using the same method as in Example 3. In addition, "human_SI" represents the gene expression based on the total RNA of a human small intestine, and DuSMFs, DuSPFs, ILSMFs, and ILSPFs represent the gene expression of a fibroblast near the mucosa and a fibroblast near the chorion under the duodenal mucosa, duodenal peritoneum, ileac mucosa, and ileac peritoneum respectively.

The results shown in FIG. 11 have revealed that a fibroblast-like cell derived from an intestinal structure expresses all the genes that are expressed by an intestinal tract and a site-specific cell constituting an intestinal tract, and shown in the same drawing. In addition, it has been revealed that, with regard to the genes expressed by an intestinal tract and a site-specific cell constituting an intestinal tract, a fibroblast-like cell derived from an intestinal structure is much different in the gene expression pattern from the various kinds of fibroblasts in an organism. These results suggest that differently from various kinds of fibroblasts in an organism, a fibroblast-like cell derived from the intestinal structure can help to maintain and/or proliferate a cell such as an intestinal epithelial cell constituting an intestinal tract.

### [Example 9] Gene analysis 2 of fibroblast-like cell derived from intestinal structure

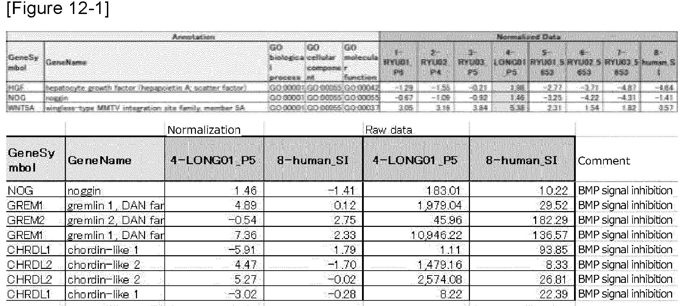

A fibroblast-like cell and an intestinal epithelial cell-like cell that were derived from an intestinal structure, and produced using the same method as in Example 3 were analyzed for the gene expression of an intestinal tract and a site-specific cell constituting an intestinal tract. Furthermore, the gene expression based on the total RNA of a human small intestine was analyzed. The results are shown in FIG. 12-1. In FIG. 12-1, "RYU01", "RYU02", and "RYU03" each represent the gene expression of an intestinal epithelial cell-like cell derived from an intestinal structure, and produced using the same method as in Example 3. In addition, "LONG01" represents the gene expression of a fibroblast-like cell derived from an intestinal structure, and produced using the same method as in Example 3. In addition, "human_SI" represents the gene expression based on the total RNA of a human small intestine.

The results shown in FIG. 12-1 have revealed that a fibroblast-like cell derived from an intestinal structure expresses each of NOG, HGF, WNT5A, and GREM1, the genes that are expressed by an intestinal tract and a site-specific cell constituting an intestinal tract, and shown in the same drawing. Here, it is known that NOG, WNT5A, and GREM1 each act as a factor that maintains an intestinal epithelial cell (see Hans Clevers et al., Gastroenterology, 2012; 143: 1518-1529, "Redundant Sources of Wnt Regulate Intestinal Stem Cells and Promote Formation of Paneth" ). In addition, it is known that HGF contributes to maintaining the homeostasis of a colonic epithelial cell. Accordingly, these results suggest that a fibroblast-like cell derived from an intestinal structure can help to maintain and/or proliferate a cell such as an intestinal epithelial cell-like cell constituting an intestinal tract.

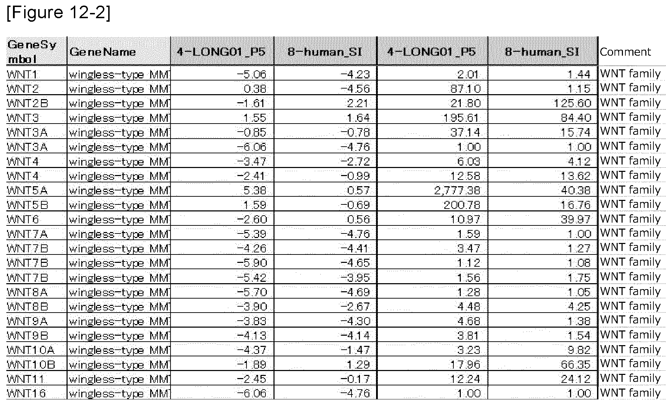

In addition, the gene expression of the WNT family that are different kinds of signaling proteins was analyzed with respect to a fibroblast-like cell and an intestinal epithelial cell-like cell that were derived from an intestinal structure, and produced using the same method as in Example 3. The results are shown in FIG. 12-2. In FIG. 12-2, "LONG01" represents the gene expression of a fibroblast-like cell derived from an intestinal structure, and produced using the same method as in Example 3. In addition, "human_SI" represents the gene expression based on the total RNA of a human small intestine.

The results shown in FIG. 12-2 have revealed that a fibroblast-like cell derived from an intestinal structure has a difference in the gene expression pattern of the WNT family from a human small intestine. This result suggests that a fibroblast-like cell derived from an intestinal structure is different from a human small intestine, i.e., a naturally-occurring fibroblast.

### [Example 10] Hierarchical clustering by comprehensive gene analysis of fibroblast-like cell derived from intestinal structure

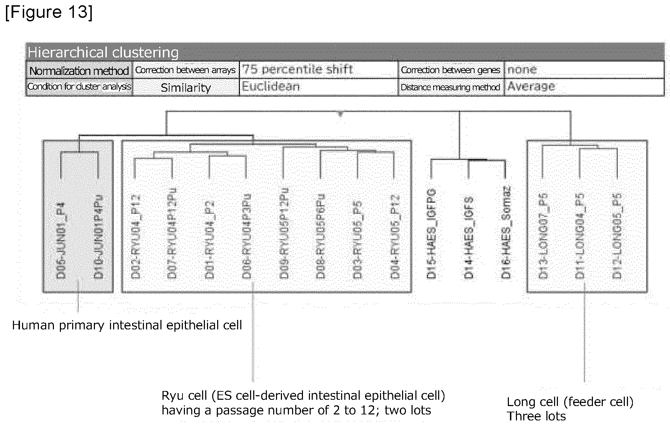

Hierarchical clustering by comprehensive gene expression analysis was performed on a fibroblast-like cell and an intestinal epithelial cell-like cell that were produced using the same method as in Example 3, and derived from an intestinal structure. Furthermore, hierarchical clustering by comprehensive gene expression analysis was performed also on a primary intestinal epithelial cell collected from a human. The results are shown in FIG. 13. In this regard, in FIG. 13, "Ryu cell" and "Long cell" represent an intestinal epithelial cell-like cell and a fibroblast-like cell respectively that were derived from an intestinal structure.

Referring to the results shown in FIG. 13, the Ryu cell that is an intestinal epithelial cell cocultured with the Long cell that it a fibroblast-like cell derived from an intestinal structure did not exhibit correlation between the passage number and the gene expression pattern, compared with a human primary intestinal epithelial cell. This result strongly suggests that, in the Ryu cell that is an intestinal epithelial cell-like cell cocultured with the Long cell that is a fibroblast-like cell derived from an intestinal structure, dedifferentiation depending on the passage number did not occur (that is, the Ryu cell was maintained).

### [Example 11] Barrier properties of cell sheet of intestinal epithelial cell-like cell derived from an intestinal structure

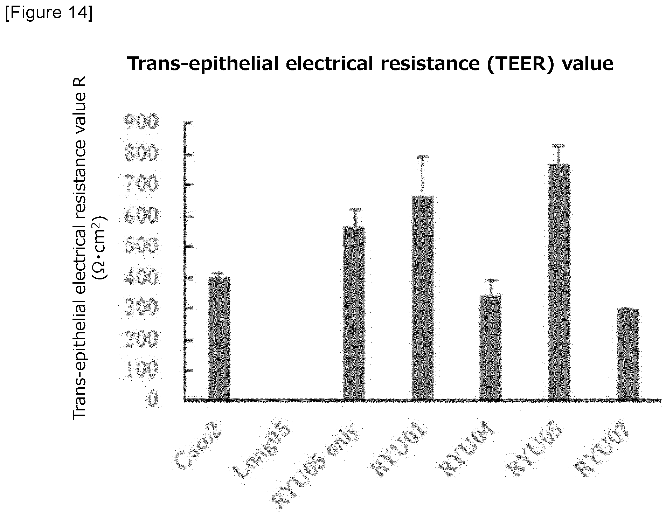

A fibroblast-like cell and an intestinal epithelial cell-like cell that were derived from an intestinal structure, and produced using the same method as in Example 3 were cocultured two-dimensionally for 7 days by culturing the fibroblast-like cell on Transwell for 5 days to make the cell confluent, and then further seeding the intestinal epithelial cell-like cell at 6.0 × 10⁴/well. For the culture, a D-MEM medium supplemented with FBS at 15% was used. After completion of the culture, the coculture product obtained was used as a cell sheet. Then, using a chopstick type electrode, the barrier properties (a trans-epithelial electrical resistance value) were verified by reading the resistance value at the point of time when 20 seconds elapsed after the electrode was inserted into a cup. The results are shown in FIG. 14. In this regard, in FIG. 14, "Caco2" is a cell sheet of a Caco-2 cell that is commonly used as a drug development tool. The Caco2 used was that which was seeded at 6.0 × 10⁴/well on Transwell, and, cultured for 11 days. "Long05" represents a cell sheet of a fibroblast-like cell derived from an intestinal structure. In addition, "RYU01" , "RYU04" , "RYU05" , and "RYU07" refer to different intestinal epithelial cell-like cells produced using different ES cells. "RYU05 only" is a sample obtained by causing a cell death to only a fibroblast-like cell layer to a degree to which a sheet was not detached, in which the cell death was caused using puromycin 2 days before measurement of the barrier properties (a trans-epithelial electrical resistance value).

The results shown in FIG. 14 have revealed that a cell sheet of each of "RYU05 only", "RYU01", "RYU04", "RYU05", and "RYU07" obtained by coculturing a fibroblast-like cell and an intestinal epithelial cell-like cell that were derived from an intestinal structure had barrier properties (a trans-epithelial electrical resistance value) approximately equal to or higher than the barrier properties of a cell sheet of the Caco-2 cell that is commonly used as a drug development tool.

### [Example 12] Shape and transporter expression 1 of coculture product of fibroblast-like cell and intestinal epithelial cell-like cell that are derived from intestinal structure

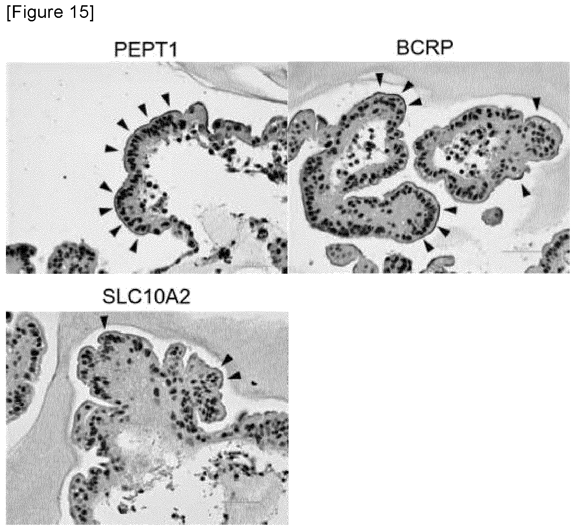

A fibroblast-like cell and an intestinal epithelial cell-like cell that were produced using the same method as in Example 3, and derived from an intestinal structure were cocultured. Specifically, an intestinal epithelial cell-like cell having a passage number of 12 and a fibroblast-like cell having a passage number of 7 were cocultured two-dimensionally for 7 days. For the culture, an ESTEM-HE medium was used. After completion of the culture, the shape of the coculture product obtained was observed under a microscope, and furthermore, the coculture product was embedded in IP gel. The coculture product embedded and fixed in the IP gel was sliced to obtain a coculture product section. The section obtained was immunostained using an antibody against each of PEPT1, BCRP, and SLC10A2, transporters of an intestinal epithelial cell. The expression of each was verified. The results are shown in FIG. 15. In this regard, in each photograph in FIG. 15, the scale bar denotes 50 *µ* m.

The photographs shown in FIG. 15 have verified that a coculture product of a fibroblast-like cell and an intestinal epithelial cell-like cell that were derived from an intestinal structure has a similar shape to the shape of the intestinal epithelium, and that a transporter is localized on the lumen side (i.e., Apical side) in an intestinal epithelium of an organism in the same manner as in an intestinal epithelium of an organism. These photographs suggest that a coculture product of a fibroblast-like cell and an intestinal epithelial cell-like cell that were derived from an intestinal structure had similarity in function to the mucosal structure of an intestine.

### [Example 13] Expression of transporter and metabolic enzyme of fibroblast-like cell and intestinal epithelial cell-like cell that are derived from intestinal structure

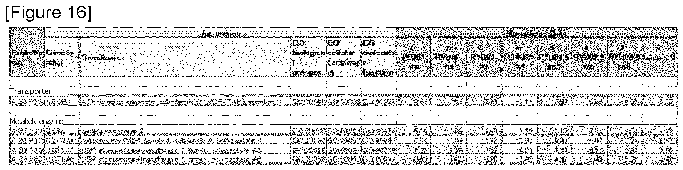

A fibroblast-like cell and an intestinal epithelial cell-like cell that were produced using the same method as in Example 3, and derived from an intestinal structure were cocultured. Specifically, an intestinal epithelial cell-like cell having a passage number of 12 and a fibroblast-like cell having a passage number of 7 were cocultured two-dimensionally for 7 days. For the culture, an ESTEM-HE medium was used. With respect to a fibroblast-like cell and an intestinal epithelial cell-like cell that had the respective passage numbers, the expression of ABCB1, a transporter of an intestinal epithelial cell, and the expression of the metabolic enzymes CES2, CYP3A4, UGT1A8, and UGT1A6 were verified. The results are shown in FIG. 16. In this regard, in FIG. 16, "RYU" represents the gene expression of an intestinal epithelial cell-like cell derived from an intestinal structure, "LONG" represents the gene expression of a fibroblast-like cell derived from an intestinal structure, and "human_SI" represents the gene expression based on the total RNA of a human small intestine. In addition, "5653" means that the cell was treated with puromycin.

The results shown in FIG. 16 have verified that, in "RYU" that is an intestinal epithelial cell-like cell derived from an intestinal structure, the transporter ABCB1 and the metabolic enzymes CES2, UGT1A8, and UGT1A6 were expressed. In addition, in FIG. 16, any of the RYUS6S3' s treated with puromycin that is an antibiotic having cytotoxicity survived, and in addition, exhibited a high CYP3A4-transcriptional level, thus verifying a high CYP responsiveness to puromycin. These results have verified that "RYU" , an intestinal epithelial cell-like cell derived from an intestinal structure, has a high drug metabolizing activity.

### [Example 14] Verification of division of intestinal epithelial cell-like cell in coculture product of fibroblast-like cell and intestinal epithelial cell-like cell

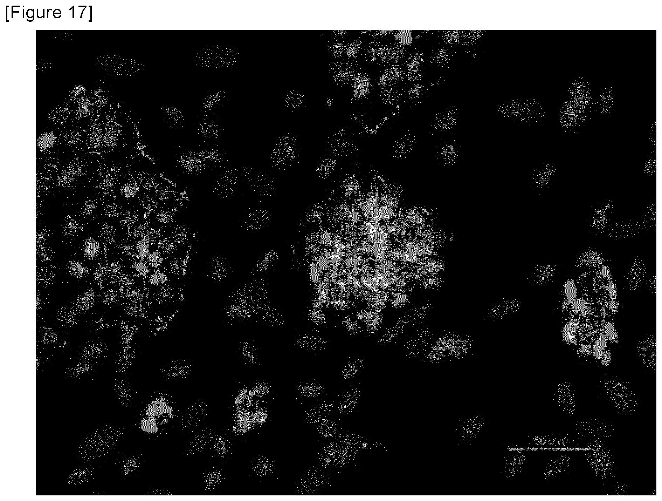

A fibroblast-like cell and an intestinal epithelial cell-like cell that were produced using the same method as in Example 3, and derived from an intestinal structure were cocultured. Specifically, an intestinal epithelial cell-like cell having a passage number of 5 and a fibroblast-like cell having a passage number of 12 were cocultured two-dimensionally for 7 days. For the culture, an ESTEM-HE medium was used. After completion of the culture, the coculture product was immunostained using an antibody against Ki-67, a marker for cell proliferation. The results are shown in FIG. 17. In this regard, in each photograph in FIG. 17, the scale bar denotes 50 *µ* m.

The photograph FIG. 17 have revealed that Ki-67, a marker for cell proliferation, is present nonlocally. This result suggests that division of a cell does not locally arise from a single cell, and arises nonlocally. This result suggests at least two possibilities. A first possibility is that the result in FIG. 17 suggests the characteristics of allowing a fibroblast-like cell (i.e., a feeder cell according to the present disclosure) derived from an intestinal structure to proliferate an object cell. A second possibility is that FIG. 17 suggests that an intestinal epithelial cell-like cell derived from an intestinal structure divides and proliferates. That is, this suggests that an intestinal epithelial cell has proliferated through division, although it is usually considered that the intestinal epithelial cell is generated only through differentiation from an intestinal epithelium stem cell (Lgr5-positive stem cell), not generated through division and proliferation of an intestinal epithelial cell after differentiation.

### [Example 15] Verification of mucosa-forming capability of fibroblast-like cell and intestinal epithelial cell that are derived from intestinal structure

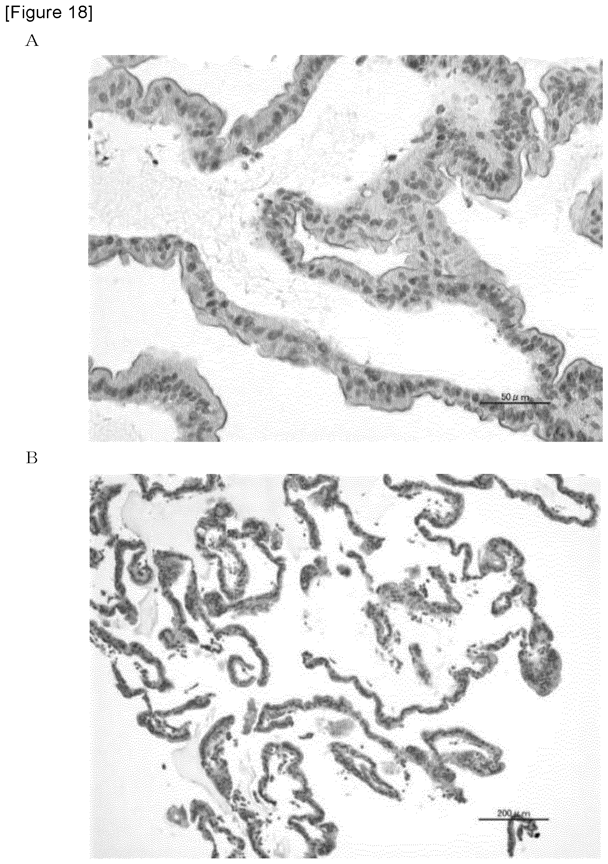

A fibroblast-like cell and an intestinal epithelial cell-like cell that were produced using the same method as in Example 3, and derived from an intestinal structure were cocultured. Specifically, an intestinal epithelial cell-like cell having a passage number of 7 and a fibroblast-like cell having a passage number of 12 were cocultured two-dimensionally for 3 days. For the culture, an ESTEM-HE medium was used. After completion of the culture, a coculture product was embedded in IP gel, and stained with Alcian blue. The results are shown in FIG. 18. In this regard, in the photographs in FIGs. 18A and 18B, the scale bars denote 50 *µ* m and 200 *µ* m respectively.

The blue coloration presented in each photograph in FIG. 18 shows the presence of a mucilaginous substance, and suggests the presence of a goblet cell that is classified as an intestinal epithelial cell. This has revealed that a feeder cell according to the present disclosure has exhibited a feeder capability contributive to stable proliferation of an intestinal epithelial cell over a long period of time (a plurality of passages), and that an intestinal epithelium-like cell proliferated and maintained by a culture using a feeder cell according to the present disclosure maintains an enteric secretory capability. This result suggests that using a feeder cell according to the present disclosure makes it possible to produce a large amount of a mucilaginous substance itself or a cell that produces a mucilaginous substance, and furthermore makes it possible to produce a large amount of a cell sheet having such a mucilaginous substance. It is known that almost all of the mucus secreted by an animal contains mucin, and that most mucosa in the body of an animal is covered with mucin. Thus, a feeder cell according to the present disclosure is expected to be applied in medical fields, particularly medical fields related to mucus and mucosa. In addition, a Caco-2 cell has hitherto been commonly used as a drug development tool, but the Caco-2 cell commonly does not have the capability to secrete a mucilaginous substance. Thus, it is also suggested that using a feeder cell according to the present disclosure makes it possible to provide an assay system closer to an organism than the Caco-2 cell.

### [Example 16] Verification 1 of feeder capability of fibroblast-like cell derived from intestinal structure

The feeder capability of a fibroblast-like cell derived from an intestinal structure, and produced using the same method as in Example 3 was verified in comparison with a conventional feeder cell derived from a human embryonic stem cell. First, a conventional feeder cell was produced in accordance with the following procedure.

EES2, a human embryonic stem cell (hES cell), was exposed to an ROCK inhibitor (Y-27632; 10 *µ* M), then dissociated into single cells, using 0.5 mM EDTA. The cells were seeded in a 96-well plate at a concentration of 5 × 10³ cells per well.

The cells seeded were cultured in an EB medium (76% KnockOut DMEM, 20% 35 kGy-irradiated Xeno-free KnockOut Serum Replacement (XF-KSR, Life Technologies), 2 mM GlutaMAX-I, 0.1 mM non-essential amino acid (NEAA), 50 U/ml penicillin-50 *µ* g/ml streptomycin (Pen-Strep), and 50 *µ* g/ml L-ascorbic acid 2-phosphate (Sigma-Aldrich)) for 4 days to form an embryoid.

The embryoid obtained was transferred to a T25 flask coated with NMP collagen PS (NH Foods Ltd.), and cultured in an XF32 medium (85% KnockOut DMEM, 15% 35kGy-irradiated XF-KSR, 2 mM GlutaMAX-I, 0.1 mM NEAA, Pen-Strep, 50 *µ* g/ml L-ascorbic acid 2-phosphate, 10 ng/ml heregulin-1*β* (Recombinant human NRG-beta 1/HRG-beta 1 EGF domain; Fujifilm Wako Pure Chemical Corporation), 200 ng/ml recombinant human IGF-1 (LONGR3-IGF-1; Sigma-Aldrich), and 20 ng/ml human bFGF (Kaken Pharmaceutical Co., Ltd.)) for 60 to 70 days to obtain a cell derived from an hES cell. This cell has a mesenchymal cell-like property, and thus, is also referred to as an ES cell-derived mesenchymal cell.

The hES cell-derived mesenchymal cell obtained was maintained in an α -MEM medium supplemented with 10% FBS (Gibco or HyClone) and 1% Pen-Strep, whereby a feeder cell (hereinafter also referred to as a "conventional feeder cell" ) was obtained.

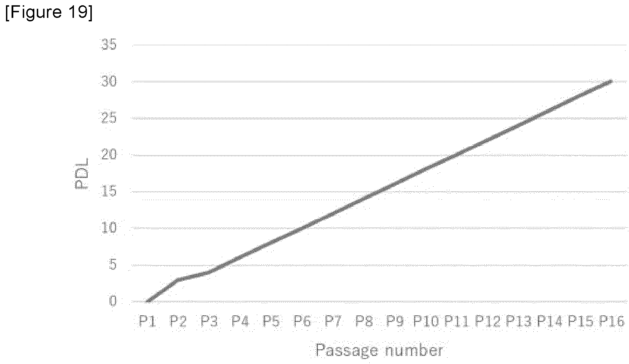

The feeder capability of a fibroblast-like cell (i.e., a feeder cell according to the present disclosure) derived from an intestinal structure, and produced using the same method as in Example 3 was verified in accordance with the following procedure. A feeder cell according to the present disclosure was cultured in a large amount to obtain a feeder cell having a passage number of 7. In the same manner as in Example 3, an intestinal epithelial cell was seeded on the resulting feeder cell, and cultured to repeat a passage. A PDL (Population doubling level: the ordinate) at each passage number of an intestinal epithelial cell that repeated a passage is shown in FIG. 19.

The results shown in FIG. 19 have verified that the feeder cell according to the present disclosure exhibited a stable proliferative capability of the object cell (intestinal epithelial cell) at any of the passage numbers of 1 to 16 (P1 to 16). That is, it has been revealed that a feeder cell according to the present disclosure exhibits a feeder capability contributive to stable proliferation of an object cell over a long period of time (a plurality of passages).

### [Example 17] Verification 2 of feeder capability of fibroblast-like cell derived from intestinal structure

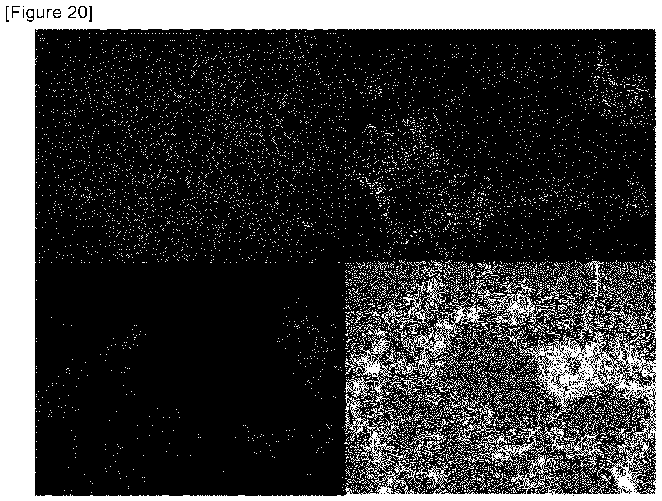

The feeder capability (i.e., a feeder cell according to the present disclosure) of a fibroblast-like cell derived from an intestinal structure, and produced using the same method as in Example 3 was verified in comparison with a conventional feeder cell derived from a human embryonic stem cell. Specifically, a conventional feeder cell produced using the same method as in Example 16 was first prepared. Then, a feeder cell according to the present disclosure and a conventional feeder cell were each seeded in a 24-well plate at a concentration of 5 × 10⁴ cells per well, and cultured in an α -MEM medium supplemented with FBS at 10% and penicillin-streptomycin at 1%. After 1 day, an intestinal epithelial cell-like cell (having a passage number of 3) derived from an intestinal structure was seeded at a concentration of 1.0 × 10⁵ per well. For the culture, an ESTEM-HE medium was used. The culture product after being cultured (for 1 passage) for 7 days was immunostained using an antibody against CDX2 and Villin, markers for an intestinal epithelial cell. The results obtained by culturing an intestinal epithelial cell-like cell using a conventional feeder cell are shown in FIG. 20. In this regard, in FIG. 20, the upper left photograph shows a photograph of immunostaining of CDX2, the upper right shows a photograph of immunostaining of Villin, the lower left shows a photograph of immunostaining by DAPI, and the lower right shows a photomicrograph of an intestinal epithelial cell-like cell after culture.

According to the results shown in FIG. 20, a very large oblate cell was found in the center in a case where an intestinal epithelial cell-like cell was cultured using a conventional feeder cell. In addition, the expression of Villin was verified, but the expression of CDX2 was little verified. This result suggests that, in a case where a conventional feeder cell is used, the expression of CDX2, which is usually expressed in an intestinal epithelial cell, is lost, and that the characters of an intestinal epithelial cell-like cell are changed in a short period of time (a small passage number). In this regard, although the results are not shown, it has been verified that, in a case where an intestinal epithelial cell-like cell was cultured using a feeder cell according to the present disclosure, the expression of CDX2 and Villin was verified with the same passage number, and the characters of an intestinal epithelial cell were maintained.

### [Example 18] Maintenance of chondrocyte by fibroblast-like cell derived from intestinal structure

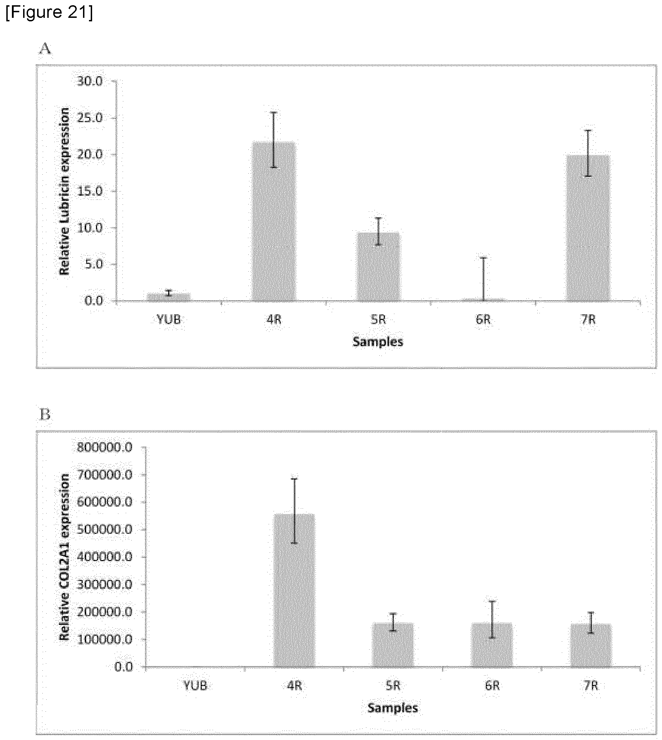

The feeder capability (maintenance of a chondrocyte) of each of four different fibroblast-like cells (i.e., feeder cells according to the present disclosure) derived from an intestinal structure, having a passage number 7, and produced using the same method as in Example 3 was verified. Specifically, the expression of lubricin and COL2A1 was verified, using qRT-PCR, each of a chondrocyte (YUB) collected from a human child and a chondrocyte cultured together with each of the above-described four kinds of fibroblast-like cells. The results are shown in FIG. 21. In addition, in the Table, "YUB" represents a chondrocyte collected from a child specimen, and "4R" to "7R" mean chondrocytes cultured together with the respective fibroblast-like cells (in this regard, no data was obtained from "6R" in FIG. 21A).

The results in FIG. 21 have verified that, in the chondrocytes 4R to 7R cultured together with a feeder cell according to the present disclosure, lubricin and COL2A1 were highly expressed, compared with YUB, a chondrocyte collected from a human child. Here, lubricin is a lubricant substance that is highly expressed in a synoviocyte known as a feeder cell of a chondrocyte, and COL2A1 is a lubricant substance that is highly expressed in a chondrocyte. In addition, it is known that a chondrocyte is a cell prone to dedifferentiation. Accordingly, the above-described results were conceivably obtained as follows: in YUB, the chondrocyte was dedifferentiated and lost its original property, and thus, the expression of COL2A1 as a matter of course and the expression of lubricin were almost not recognized; but, in 4R to 7R, the feeder cells according to the present disclosure achieved a synoviocyte-like function, allowed lubricin to be highly expressed, and furthermore allowed the original property of the chondrocyte to be maintained, allowing COL2A1 to be highly expressed. These results suggest that a feeder cell according to the present disclosure has a feeder capability for a chondrocyte.

### [Example 19] Marker verification by gene expression profile and immunostaining of feeder cell according to the present disclosure

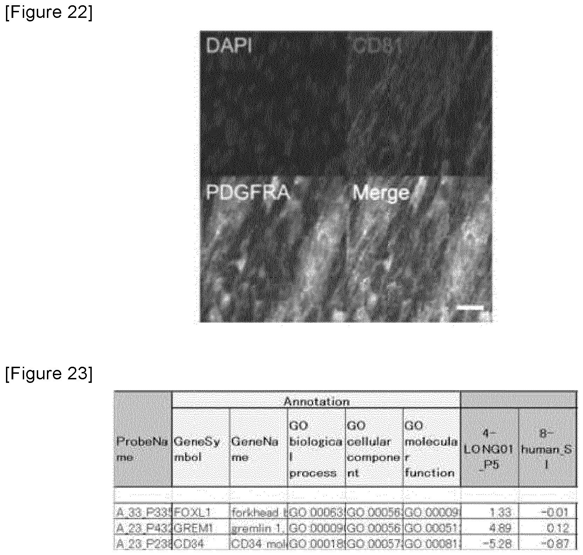

The cell marker for a fibroblast-like cell (i.e., a feeder cell according to the present disclosure) derived from an intestinal structure, and produced using the same method as in Example 3 was verified using immunostaining. Specifically, a fibroblast-like cell cultured was embedded in IP gel to produce a specimen. The protein expression on the cell surface of each of PDGFRA and CD81 was stained using PDGF Receptor α (D1E1E) XP (registered trademark) Rabbit mAb manufactured by Cell Signaling Technology, Inc. and Anti-CD81, Mouse-Mono (M38) manufactured by EXBIO respectively as primary antibodies. The results are shown in FIG. 22.

In addition, the expression of each of the genes, Foxl1, CD34, and GREM1, obtained through comprehensive gene expression, and subjected to normalization, was verified. The results are shown in FIG. 23.

The results shown in FIGs. 22 and 23 have verified that, in a feeder cell according to the present disclosure, each of the genes, PDGFRA, CD81, Foxl1, and GREM1 were expressed, but on the other hand, CD34 was not expressed.

### [Example 20] Production of cell sheet

Using a feeder cell according to the present disclosure, a cell sheet was produced. Specifically, a feeder cell according to the present disclosure was seeded and cultured until the cell became confluent. Then, an intestinal epithelial cell was seeded so as to be layered on the confluent feeder cell, and cultured until the intestinal epithelial cell became confluent, and formed a sheet structure with an intercellular junction. Then, the layered product of the layer of the feeder cell according to the present disclosure and the layer of the intestinal epithelial cell was detached from the culture surface to obtain a cell sheet containing the layer of the feeder cell and the layer of the intestinal epithelial cell.

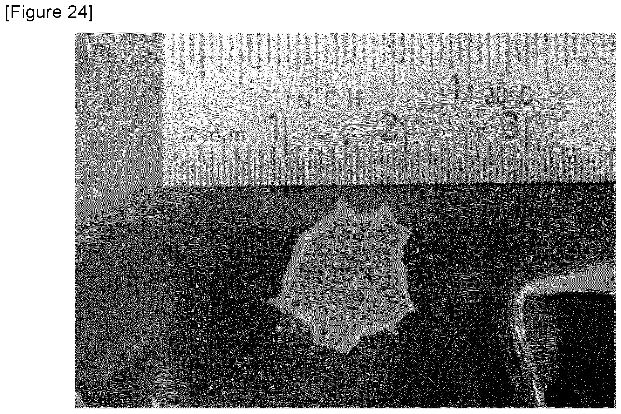

The photograph of the cell sheet obtained is shown in FIG. 24. The results shown in FIG. 24 have verified that the cell sheet according to the present disclosure had a diameter of 1 cm or more.

### Industrial Applicability

The present disclosure provides: a feeder cell for maintaining or proliferating, by a two-dimensional culture, a cell hitherto deemed to be difficult to maintain and proliferate by a two-dimensional culture; and a method of producing the feeder cell.

## Claims

1. A method of producing a feeder cell for maintaining or proliferating a cell, comprising:
(A) a step of preparing an intestinal structure produced from a pluripotent stem cell, and containing an endoderm-derived cell and a mesoderm-derived cell;
(B) a step of culturing the intestinal structure to proliferate a fibroblast-like cell derived from the intestinal structure; and
(C) a step of isolating the fibroblast-like cell to obtain a feeder cell.

2. The method according to claim 1, wherein the intestinal structure is produced using a method comprising:
(a) a step of preparing a cell culture substrate including: a substrate; and a plurality of isolated cell adhesion regions formed on the substrate, and cell non-adhesion regions that surround each of the cell adhesion regions;
(b) a step of seeding a pluripotent stem cell on the cell culture substrate; and
(c) a step of culturing, in a medium, the pluripotent stem cell seeded.

3. The method according to claim 1 or 2, further comprising (D) a step of proliferating the fibroblast-like cell isolated.

4. The method according to claim 1 or 2, wherein, in the step (B), the intestinal structure is cultured in a culture container, and at least part of the intestinal structure is in contact with the culture container.

5. The method according to claim 2, wherein a culture period in the step (c) is 60 days or more.

6. A feeder cell produced using the method according to claim 1 or 2.

7. A fibroblast-like cell which is positive for PDGFRA and CD81, and expresses Foxl1 and GREM1.

8. The fibroblast-like cell according to claim 7, which is positive for PDGFRA and CD81, is negative for CD34, and expresses Foxl1 and GREM1.

9. The fibroblast-like cell according to claim 7, which is derived from a culture product of an intestinal structure that is derived from a pluripotent stem cell or produced from a pluripotent stem cell, and contains an endoderm-derived cell and a mesoderm-derived cell.

10. The fibroblast-like cell according to claim 9, wherein the intestinal structure is produced using a method comprising:
(a) a step of preparing a cell culture substrate including: a substrate; and a plurality of isolated cell adhesion regions formed on the substrate, and cell non-adhesion regions that surround each of the cell adhesion regions;
(b) a step of seeding a pluripotent stem cell on the cell culture substrate; and
(c) a step of culturing, in a medium, the pluripotent stem cell seeded.

11. The fibroblast-like cell according to claim 9, wherein the pluripotent stem cell is human-derived.

12. The feeder cell according to claim 6 or the fibroblast-like cell according to claim 7, which is a feeder cell for maintaining or proliferating at least one cell selected from the group consisting of a small-intestine epithelial cell, large-intestine epithelial cell, hepatocyte, and chondrocyte.

13. The feeder cell according to claim 6 or the fibroblast-like cell according to claim 7, which is a feeder cell for maintaining or proliferating the at least one cell by a two-dimensional culture.

14. The feeder cell according to claim 6 or the fibroblast-like cell according to claim 7, which is a feeder cell capable of subculturing the at least one cell three times or more.

15. A cell sheet comprising the feeder cell according to claim 6 or the fibroblast-like cell according to claim 7.

16. A coculture product comprising: at least one cell selected from the group consisting of a small-intestine epithelial cell and a hepatocyte; and the feeder cell according to claim 6 or the fibroblast-like cell according to claim 7.

17. The coculture product according to claim 16, the passage number of the at least one cell is 3 or more.

18. A cell sheet comprising the coculture product according to claim 16.

19. The cell sheet according to claim 18, having a circular or generally circular shape having a diameter of 1 cm or more.

20. The cell sheet according to claim 18, comprising a layer of at least one cell selected from the group consisting of a small-intestine epithelial cell and a hepatocyte.

21. The cell sheet according to claim 20, further comprising a layer of the feeder cell.

22. An implant containing the cell sheet according to claim 15 or 18.

23. A method of maintaining or proliferating a cell, comprising a step of culturing the cell in the presence of the feeder cell according to claim 6 or the fibroblast-like cell according to claim 7.

24. The method of maintaining or proliferating a cell according to claim 23, wherein a culture for the cell is a two-dimensional culture.

25. The method of maintaining or proliferating a cell according to claim 23 or 24, wherein the cell is at least one cell selected from the group consisting of a small-intestine epithelial cell, large-intestine epithelial cell, hepatocyte, and chondrocyte.

26. The method of maintaining or proliferating a cell according to claim 23 or 24, wherein the cell and the feeder cell are derived from the same individual.

27. The method of maintaining or proliferating a cell according to claim 23 or 24, wherein the cell and the feeder cell are derived from different individuals.

28. A cell which expresses at least one gene selected from the group consisting of DCN, ACTA2, and NOG, and expresses the genes, ADAMTS19, ANO1, BLID, LOC100507053, LHX8, SFRP4, AHRR, SLC14A1, FMO3, PZP, ABCG4, EYA2, TMEM92-AS1, ANO10, EGFL6, SNCAIP, LGSN, PCDHB15, and KRTAP1-5.
